# EUROPEAN PATENT SPECIFICATION

(11) **EP 4 234 085 B1**
(45) Date of publication and mention of the grant of the patent: **25.02.2026**
(21) Application number: 23181947.5
(22) Date of filing: 16.08.2019
(51) Int. Cl.: A61B 5/15, A61B 5/154, B01L 3/02, B01L 3/00

(54) **BIOLOGICAL FLUID COLLECTION SYSTEM**
SYSTEM ZUM SAMMELN VON BIOLOGISCHEN FLÜSSIGKEITEN
SYSTÈME DE COLLECTE DE FLUIDE BIOLOGIQUE

(30) Priority: 17.08.2018 US 201862719166 P
(43) Date of publication of application: 30.08.2023
(62) Divisional of application: 19759273.6
(73) Proprietor: Becton, Dickinson and Company, Franklin Lakes, NJ 07417-1880 (US)
(72) Inventor: IVOSEVIC, Milan, Kinnelon, New Jersey 07405 (US); TORRIS, Anthony, V., Montclair, New Jersey 07042 (US); BLAKE, Alexander, James, Ridgewood, New Jersey 07450 (US)
(74) Representative: dompatent

(56) References cited:
- EP-A1- 1 568 321
- EP-A2- 1 293 162
- EP-A2- 1 362 608
- WO-A1-2013/059429
- WO-A2-2016/205779
- US-A- 2 688 325
- US-A1- 2003 229 315
- US-A1- 2004 143 226
- US-A1- 2014 305 196
- US-A1- 2017 059 550
- US-A1- 2017 216 835

## Description

### CROSS-REFERENCE TO RELATED APPLICATION

The present application claims priority to United States Provisional Application Serial No. 62/719,166, filed August 17, 2018, entitled "Biological Fluid Collection System and Stabilization Assembly'.

### BACKGROUND OF THE INVENTION

### 1. Field of the Disclosure

The present disclosure relates generally to a biological fluid collection systems. More particularly, the present disclosure relates to a collection module for collecting a small sample of blood and dispensing a portion of the sample into a device for analyzing the sample such as a point-of-care or a near-patient-testing device and blood collection devices for collecting of arterial blood samples to be delivered to a blood gas analyzer. The present invention relates to a fluid collection system according to the preamble of claim 1. Such a fluid collection system is known from WO 2013/059429 A1.

### 2. Description of the Related Art

A need exists for a device which enables collection of a micro-sample, such as less than 1.0 milliliters of collected sample for analysis, for patient point-of-care and blood gas analysis applications. Current devices require conventional sample collection and the subsequent use of a large syringe or pipette to transfer a small blood sample to a point-of-care cartridge or instrument receiving port. Such an open system approach results in an increased blood exposure risk for personnel performing the testing, as well as the collection of excess specimen required for a specified test procedure.

It is therefore desirable to have a blood sample collection and dispensing tool for point-of-care blood gas analysis applications which incorporates conventional automatic blood draw and includes a novel controlled sample transfer capability while minimizing exposure risk.

A need also exists for reducing the number of workflow steps in an arterial blood gas collection procedure.

### SUMMARY OF THE INVENTION

A biological fluid collection system including a collection module for collecting a small sample of blood and dispensing a portion of the sample into a device for analyzing the sample such as a point-of-care or a near-patient-testing device such as blood collection devices for collecting of venous and arterial blood samples are disclosed.

In accordance with the invention there is provided a fluid collection system comprising the features of claim 1. Preferred embodiments of the present invention are defined in the dependent claims.

A device of the present disclosure has the following advantages over conventional arterial blood gas (ABG) collection kits: (1) ergonomic design with designated touch points; (2) thin wall needle technology that allows a small gage needle while still maintaining high flow rate resulting in fast fill time; (3) push-button safety shield that could be singlehanded activated by simple push of the button after the collection while not obscuring a user's view during the procedure; (4) integrated air venting/venting cap capability which allows removal of trapped air bubbles by simple expelling during fill or after collection; and (5) utilizes a safety sleeve with guard and push button that prevents accidental safety shield activation during the device transport.

### BRIEF DESCRIPTION OF THE DRAWINGS

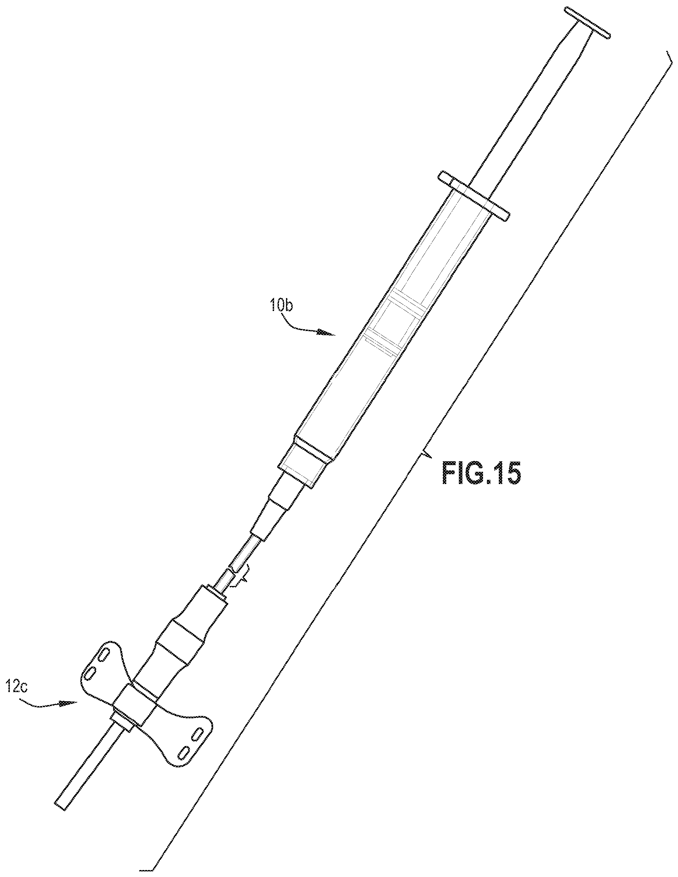

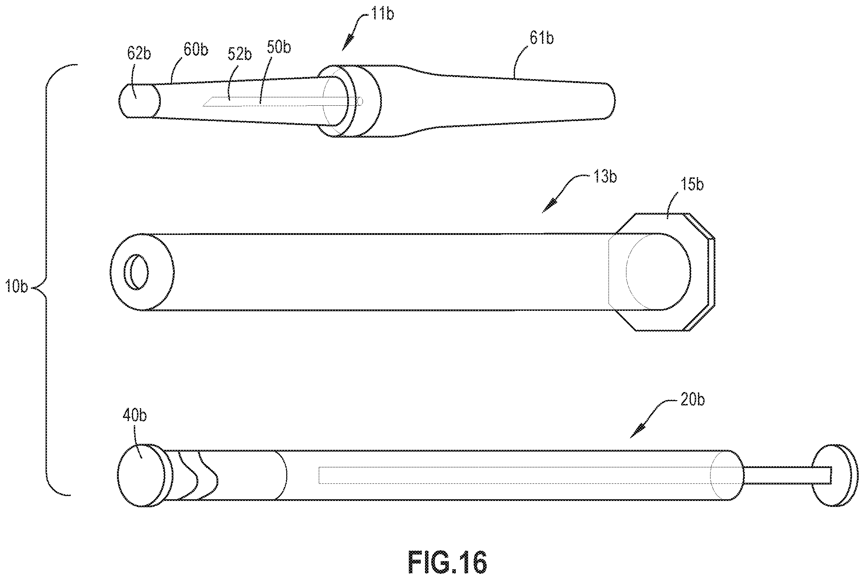

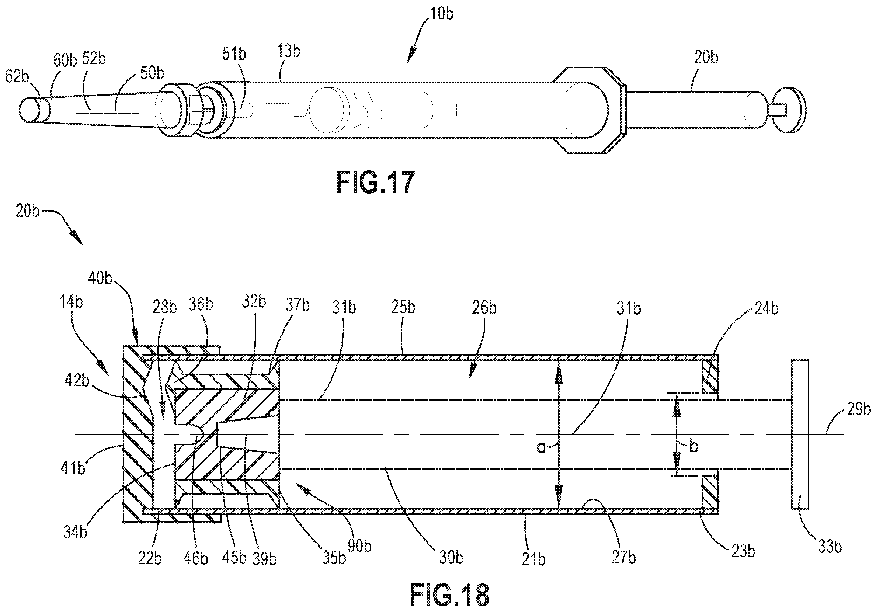

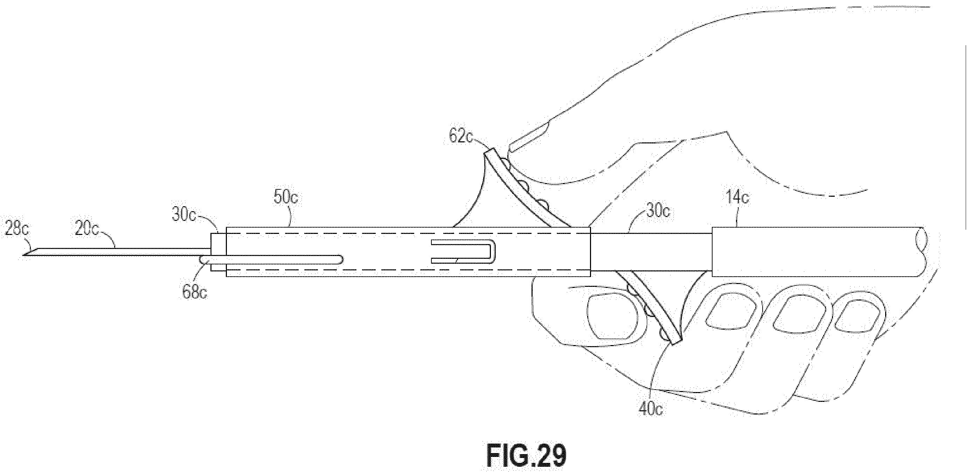

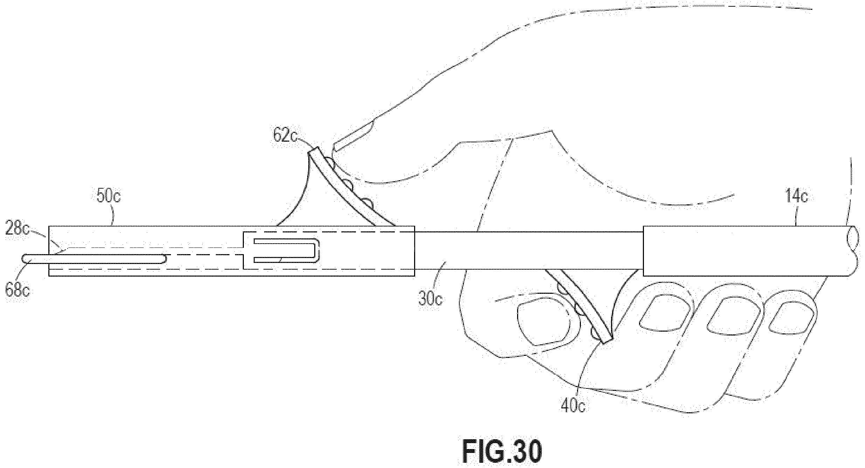

The above-mentioned and other features and advantages of this disclosure, and the manner of attaining them, will become more apparent and the disclosure itself will be better understood by reference to the following descriptions of embodiments of the disclosure taken in conjunction with the accompanying drawings. Without limitations, the invention is best illustrated by figures 15 to 30.
**FIG. 1** is a perspective view of a biological fluid collection system in accordance with another embodiment of the present invention.
**FIG. 2** is a cross-sectional side elevation view of a collection module with a cap in accordance with an embodiment of the present invention.
**FIG. 3** is a cross-sectional side elevation view of a collection module with a deformable portion in an initial position in accordance with an embodiment of the present invention.
**FIG. 4** is a cross-sectional side elevation view of a biological fluid collection system with a lock in a locked position in accordance with an embodiment of the present invention.
**FIG. 5** is a cross-sectional side elevation view of a biological fluid collection system with a lock in an unlocked position in accordance with an embodiment of the present invention.
**FIG. 6** is a cross-sectional perspective view of a collection module with a deformable portion in an initial position adjacent a point-of-care testing device in accordance with an embodiment of the present invention.
**FIG. 7** is a cross-sectional perspective view of a collection module with a deformable portion in a deformed position adjacent a point-of-care testing device in accordance with an embodiment of the present invention.
**FIG. 8** is a perspective view of a collection module in accordance with an embodiment of the present invention.
**FIG. 9** is an exploded perspective view of a needle assembly having a hinged safety shield in accordance with an embodiment of the present invention.
**FIG. 10** is an assembled perspective view of the needle assembly of **FIG. 9** in the retracted position.
**FIG. 11** is a cross-sectional side view of the needle assembly of **FIG. 10****.**
**FIG. 12** is a perspective view of the needle assembly of **FIG. 10** in the extended position.
**FIG. 13** is a perspective view of a biological fluid collection system in accordance with another embodiment of the present invention.
**FIG. 14** is a perspective view of a biological fluid collection device inserted into a tube holder in accordance with an embodiment of the present invention.
**FIG. 15** is a perspective view of a fluid collection system in accordance with another embodiment of the present invention.
**FIG. 16** is a perspective view of the components of a fluid collection assembly in accordance with an embodiment of the present invention.
**FIG. 17** is a perspective view of a fluid collection assembly in accordance with an embodiment of the present invention.
**FIG. 18** is a partial cross-sectional side view of a fluid collection cartridge as similarly shown in **FIG. 16** in accordance with an embodiment of the present invention.
**FIG. 19** is a cross-sectional side view of the fluid collection assembly as shown in **FIG. 18** during the insertion of a fluid collection cartridge into the holder in accordance with an embodiment of the present invention.
**FIG. 20** is a cross-sectional side perspective view of the fluid collection assembly as shown in **FIG. 17** during the insertion of the fluid collection cartridge into the holder in accordance with an embodiment of the present invention.
**FIG. 21** is a cross-sectional side perspective view of the fluid collection assembly as shown in **FIG. 17** after priming with a fluid treatment additive and prior to collection of a fluid sample in accordance with an embodiment of the present invention.
**FIG. 22** is a cross-sectional side view of the fluid collection assembly as shown in **FIG. 19** after priming with a fluid treatment additive and during the removal of the plunger rod in accordance with an embodiment of the present invention.
**FIG. 23** is a perspective view of the fluid collection assembly during fluid collection in accordance with an embodiment of the present invention.
**FIG. 24** is a cross-sectional side view of the fluid collection assembly as shown in **FIG. 23** upon completion of collection of a fluid sample in accordance with an embodiment of the present invention.
**FIG. 25** is a plan view of the fluid collection cartridge showing the direction of agitation after collection of a fluid sample in accordance with an embodiment of the present invention.
**FIG. 26** is a cross-sectional side view of the fluid collection cartridge showing the mixing dynamics after collection of a fluid sample in accordance with an embodiment of the present invention.
**FIG. 27** is a perspective side view of the fluid collection cartridge with a luer adapter in preparation for transportation and testing in accordance with an embodiment of the present invention.
**FIG. 28** is a perspective view of a blood collection set in accordance with the present invention.
**FIG. 29** is a side plan view of the blood collection set showing the outer shield in a retracted position.
**FIG. 30** is a side plan view of the blood collection set showing the outer shield in an extended position.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

The following description is provided to enable those skilled in the art to make and use the described embodiments contemplated for carrying out the invention. Various modifications, variations, and alternatives, however, will remain readily apparent to those skilled in the art. Any and all such modifications, variations, and alternatives are intended to fall within the scope of the present invention

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

The present disclosure provides a biological fluid collection system that includes a power source for a collection module that receives a sample and provides flow-through blood stabilization technology and a precise sample dispensing function for point-of-care and near patient testing applications. A collection module of the present disclosure is able to effectuate distributed mixing of a sample stabilizer within a blood sample and dispense the stabilized sample in a controlled manner. In this manner, a biological fluid collection system of the present disclosure enables blood micro-sample management, e.g., passive mixing with a sample stabilizer and controlled dispensing, for point-of-care and near patient testing applications.

Advantageously, a biological fluid collection system of the present disclosure provides a consistent blood sample management tool for point-of-care and near patient testing applications, automatic blood draw, passive mixing technology, and controlled small sample dispensing capability to point-of-care cartridge and standard luer interfaces with near patient testing receiving ports.

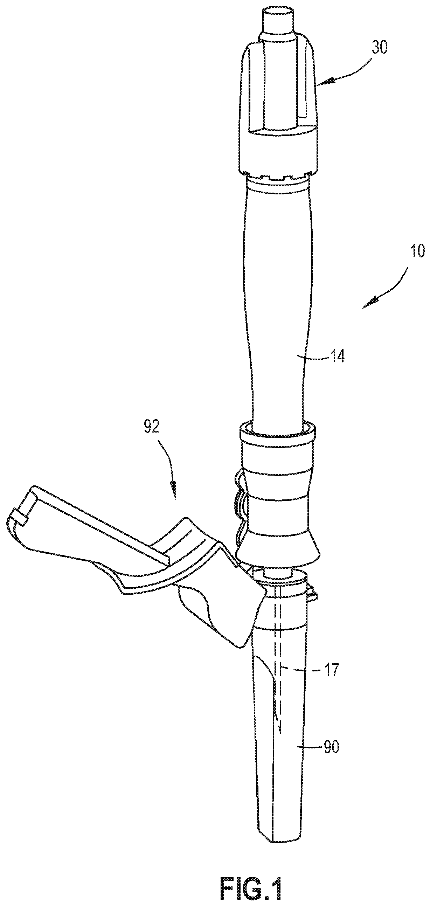

Figs. 1-8 illustrate exemplary embodiments of a biological fluid collection system 10 of the present disclosure that is adapted to receive a biological fluid sample, such as a blood sample 12. In one embodiment, the biological fluid collection system 10 of the present disclosure includes a collection module 14 that is adapted to receive a blood sample 12 and a power source 16 that is removably connectable with the collection module 14. A power source of the present disclosure provides a user activated vacuum source for drawing a biological fluid sample within a collection module 14. In one embodiment, a portion of the collection module 14 includes a cannula 17 for obtaining a blood sample 12 from a patient into the collection module 14.

Referring to Figs. 1-8, in one embodiment, the collection module 14 of the present disclosure is adapted to receive a biological fluid sample, such as a blood sample 12, and includes a housing 20, a mixing chamber 22, a sample stabilizer 24, a collection chamber 26, a closure 28, and a cap 30.

In one embodiment, the housing 20 of the collection module 14 includes an inlet port 32 and an outlet port 34. The inlet port 32 and the outlet port 34 are in fluid communication via a passageway 36 extending therebetween.

The mixing chamber 22 and the collection chamber 26 are provided in fluid communication with the passageway 36. The mixing chamber 22 and the collection chamber 26 are positioned such that a biological fluid sample, such as a blood sample 12, introduced into the inlet port 32 of the collection module 14 will first pass through a sample stabilizer 24, then the blood sample 12 and the sample stabilizer 24 pass through the mixing chamber 22, and subsequently the sample 12 with the sample stabilizer 24 properly mixed therein flow into the collection chamber 26, prior to reaching the outlet port 34 of the collection module 14. In this way, the blood sample 12 may be mixed with a sample stabilizer 24, such as an anticoagulant or other additive, provided within the collection module 14, before passing through the mixing chamber 22 for proper mixing of the sample stabilizer 24 within the blood sample 12, and then the stabilized sample is received and stored within the collection chamber 26.

In one embodiment, a sample stabilizer 24 is disposed between the inlet port 32 and the mixing chamber 22. The collection module 14 of the present disclosure provides passive and fast mixing of a blood sample 12 with the sample stabilizer 24. For example, the collection module 14 includes a mixing chamber 22 that allows for passive mixing of the blood sample 12 with an anticoagulant or another additive, such as a blood stabilizer, as the blood sample 12 flows through the mixing chamber 22.

The sample stabilizer can be an anticoagulant, or a substance designed to preserve a specific element within the blood such as, for example, RNA, protein analyte, or other element. In one embodiment, the sample stabilizer 24 is disposed between the inlet port 32 and the mixing chamber 22. In other embodiments, the sample stabilizer 24 may be disposed in other areas within the housing 20 of the collection module 14.

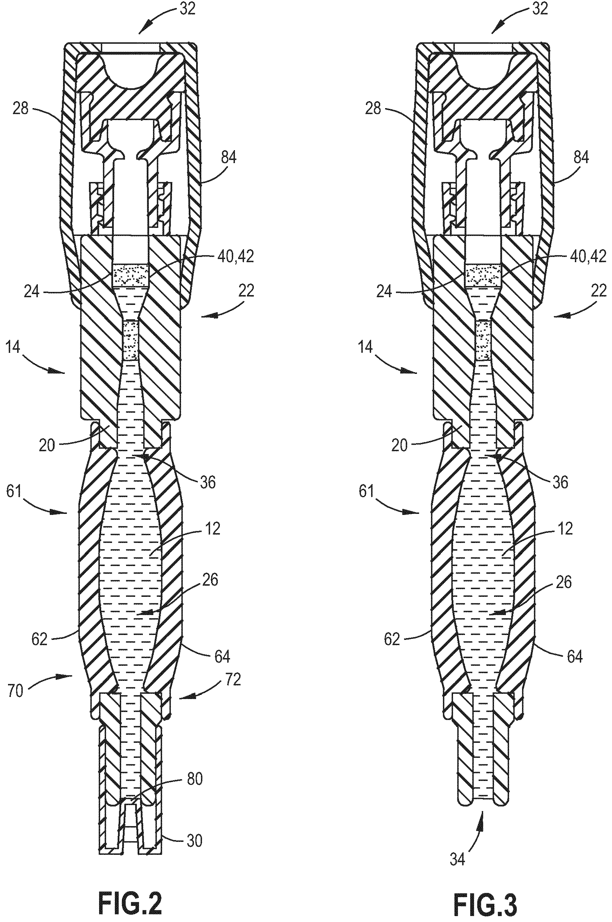

Referring to Figs. 2 and 3, in one embodiment, the collection module 14 includes a material 40 including pores 42 that is disposed between the inlet port 32 and the mixing chamber 22 and a dry anticoagulant powder that is within the pores 42 of the material 40. In this manner, the collection module 14 may include a dry anticoagulant, such as Heparin or EDTA, deposited on or within a portion of the collection module 14. In one embodiment, the material 40 is an open cell foam that contains dry anticoagulant dispersed within the cells of the open cell foam to promote the effectiveness of the flow-through mixing and anticoagulant uptake. In one embodiment, the sample stabilizer 24 is the dry anticoagulant powder.

In one embodiment, the open cell foam may be treated with an anticoagulant to form a dry anticoagulant powder finely distributed throughout the pores of the open cell foam. As the blood sample 12 enters the collection module 14, the blood sample 12 passes through the open cell foam and is exposed to the anticoagulant powder available throughout the internal pore structure of the open cell foam. In this manner, the sample 12 dissolves and mixes with the dry anticoagulant powder while passing through the material 40 or open cell foam.

The open cell foam may be a soft deformable open cell foam that is inert to blood, for example, a melamine foam, such as Basotect^{®} foam commercially available from BASF, or may consist of a formaldehyde-melamine-sodium bisulfite copolymer. The open cell foam may also be a flexible, hydrophilic open cell foam that is substantially resistant to heat and organic solvents. In one embodiment, the foam may include a sponge material.

The anticoagulant or other additive may be introduced into the open cell foam by soaking the foam in a liquid solution of the additive and water and subsequently evaporating the water forming a dry additive powder finely distributed throughout the internal structure of the foam.

The collection module 14 includes a mixing chamber 22 that allows for passive mixing of the blood sample 12 with an anticoagulant or another additive, such as a blood stabilizer, as the blood sample 12 flows through the mixing chamber 22. In one embodiment, the mixing chamber 22 is disposed between the inlet port 32 and the outlet port 34.

The internal portion of the mixing chamber 22 may have any suitable structure or form as long as it provides for the mixing of the blood sample 12 with an anticoagulant or another additive as the blood sample 12 passes through the passageway 36 of the collection module 14.

The mixing chamber 22 receives the sample 12 and the sample stabilizer 24 therein and effectuates distributed mixing of the sample stabilizer 24 within the sample 12. The mixing chamber 22 effectuates distributed mixing of the sample stabilizer 24 within the sample 12 and prevents a very high sample stabilizer concentration in any portion of the blood sample 12. This prevents underdosing of the sample stabilizer 24 in any portion of the blood sample 12. The mixing chamber 22 effectuates distributed mixing of the sample stabilizer 24 within the sample 12 so that an approximately equal amount and/or concentration of the sample stabilizer 24 is dissolved throughout the blood sample 12, e.g., an approximately equal amount and/or concentration of the sample stabilizer 24 is dissolved into the blood sample 12 from a front portion of the blood sample 12 to a rear portion of the blood sample 12.

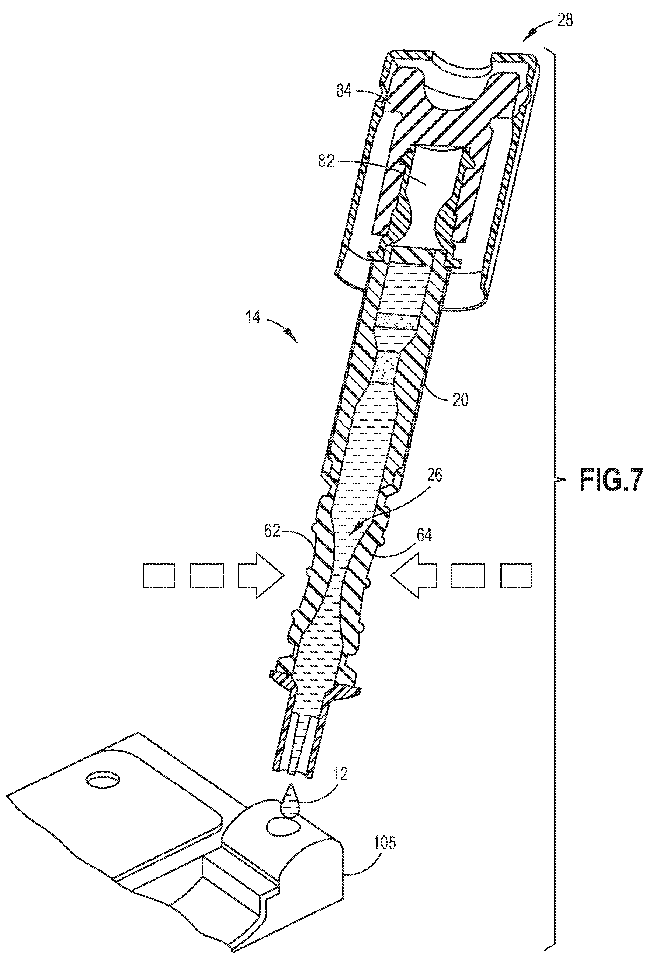

In one embodiment, the collection module 14 includes a collection chamber 26 that is disposed between the mixing chamber 22 and the outlet port 34. The collection chamber 26 includes an actuation portion 61. In one embodiment, the actuation portion 61 is transitionable between a first position (Figs. 2, 3, and 6) in which the sample 12 is containable within the collection chamber 26 and a second position (Fig. 7) in which a portion of the sample 12 is expelled from the collection chamber 26.

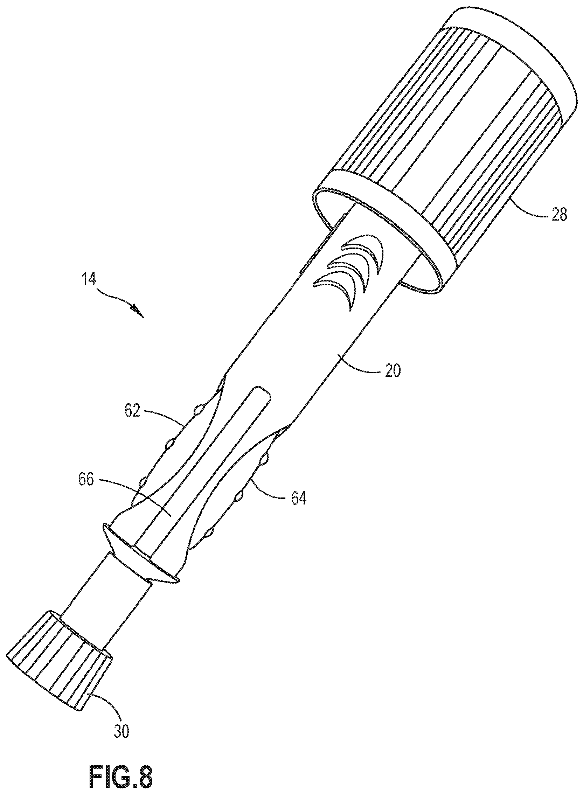

In one embodiment, the actuation portion 61 of the collection chamber 26 includes a first deformable portion 62, a second deformable portion 64, and a rigid wall portion 66 (Fig. 8) that is between the first deformable portion 62 and the second deformable portion 64. In one embodiment, the first deformable portion 62 is located on a first side 70 of the collection chamber 26 and the second deformable portion 64 is located on a second side 72 of the collection chamber 26. In one embodiment, the second side 72 of the collection chamber 26 is opposite from the first side 70 of the collection chamber 26.

In one embodiment, the first deformable portion 62 and the second deformable portion 64 are transitionable between an initial position in which the sample 12 is contained within the collection chamber 26 and a deformed position in which a portion of the sample 12 is expelled from the collection chamber 26. The first deformable portion 62 and the second deformable portion 64 are simultaneously squeezed to transition from the initial position to the deformed position.

Advantageously, by having a first deformable portion 62 and a second deformable portion 64 that can be simultaneously squeezed, a collection module 14 of the present disclosure is able to dispense more sample 12 out of the collection chamber 26 and the outlet port 34. Furthermore, in one embodiment, by having a first deformable portion 62 on a first side 70 and a second deformable portion 64 on an opposite second side 72, a collection module 14 of the present disclosure has a symmetrical design and provides a smooth straight fluid path chamber that encourages fluid attachment flow characteristics. The smooth straight fluid path chamber of the collection module 14 is without significant geometric steps in diameter and the smooth fluid pathway inhibits the formation of air pockets or bubbles.

After passing through the mixing chamber 22, the stabilized sample is directed to the collection chamber 26. The collection chamber 26 may take any suitable shape and size to store a sufficient volume of blood necessary for the desired testing, for example, 500 µl or less. In one embodiment, the collection chamber 26 is defined by a portion of the housing 20 in combination with a first deformable portion 62, a second deformable portion 64, and a rigid wall portion 66.

The first deformable portion 62 and the second deformable portion 64 may be made of any material that is flexible, deformable, and capable of providing a fluid tight seal with the housing 20. In some embodiments, the first deformable portion 62 and the second deformable portion 64 may be made of natural or synthetic rubber, and other suitable elastomeric materials. The first deformable portion 62 and the second deformable portion 64 are secured to a portion of the housing 20 such that the first deformable portion 62 and the second deformable portion 64 are transitionable between an initial position in which the sample 12 is contained within the collection chamber 26 and a deformed position in which a portion of the sample 12 is expelled from the collection chamber 26.

In another embodiment, the actuation portion 61 of the collection chamber 26 may comprise an activation member such that applying an inward pressure on an elastic portion of the actuation portion 61 forces a sample to be dispensed from the collection chamber 26. In this manner, a sample may be transferred to a device intended to analyze the sample, such as a point-of-care testing device, such as a cartridge tester or via a port while minimizing exposure of the sample. In certain configurations, the activation member may at least partially define the collection chamber 26, alternatively, the activation member may be a separate element engageable with the collection chamber 26, such as a plunger, push button, a slide, and the like. An activation member, such as that described in U.S. Patent Application Serial No. 15/065,022, filed March 9, 2016, entitled "Biological Fluid Micro-Sample Management Device", may also be used in conjunction with the present invention. In other embodiments, the actuation portion 61 of the collection chamber 26 may comprise actuation portions in accordance with actuation portions and/or deformable portions described in U.S. Patent Application Serial No. 62/634,960, filed February 26, 2018, entitled "Biological Fluid Collection Device and Collection Module'.

In one embodiment, the collection module 14 includes a cap 30 that is removably attachable to the outlet port 34 and that protectively covers the outlet port 34. In one embodiment, the cap 30 includes a venting plug 80 which allows air to pass therethrough and prevents the sample 12 from passing therethrough.

The construction of the cap 30 and venting plug 80 allows air to pass through the cap 30 while preventing the blood sample 12 from passing through the cap 30 and may include a hydrophobic filter. The venting plug 80 has selected air passing resistance that may be used to finely control the filling rate of the passageway 36 and/or the collection chamber 26 of the collection module 14. By varying the porosity of the plug, the velocity of the air flow out of the cap 30, and thus the velocity of the blood sample flow into the collection module 14, may be controlled.

In one embodiment, the collection module 14 includes a closure 28 that is engaged with the inlet port 32 of the collection module 14 to seal the passageway 36. The closure 28 protectively covers the inlet port 32. The closure 28 allows for introduction of a blood sample 12 into the passageway 36 of the housing 20 and may include a pierceable self-sealing stopper 82 with an outer shield 84 such as a Hemogard^{™} cap commercially available from Becton, Dickinson and Company.

In one embodiment, a portion of the collection module 14 includes a cannula 17 for obtaining a blood sample 12 from a patient into the collection module 14. In such embodiments, the collection module 14 includes a first removable protective cap 90 and a safety shield 92.

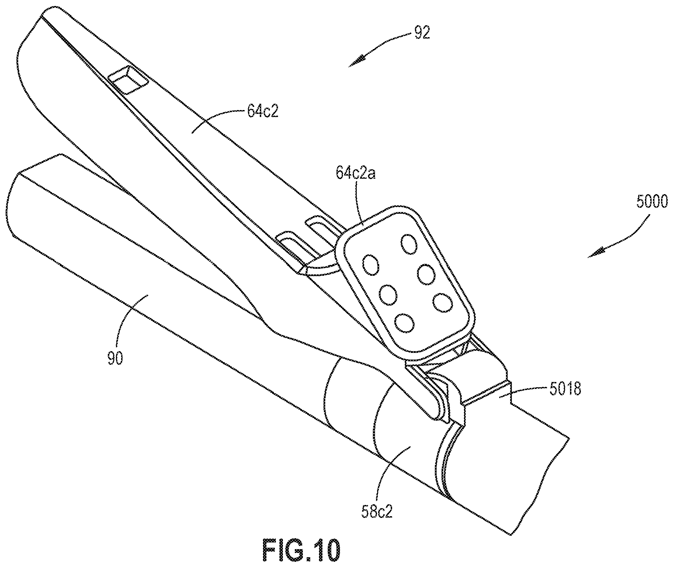

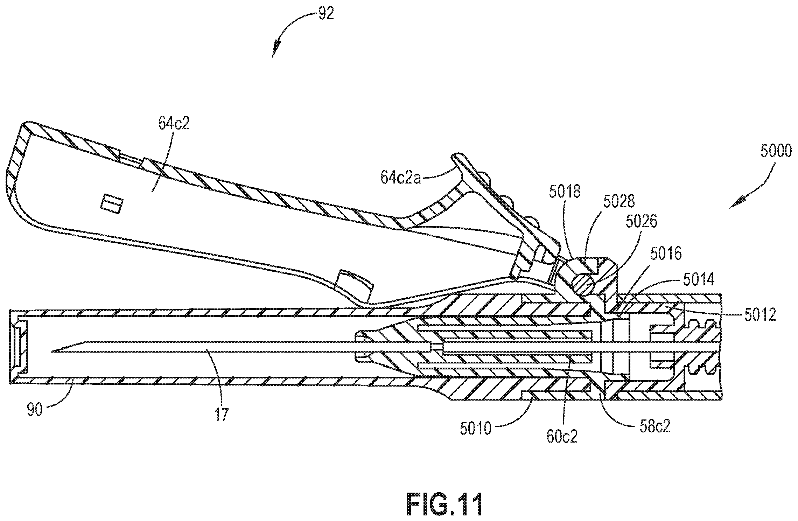

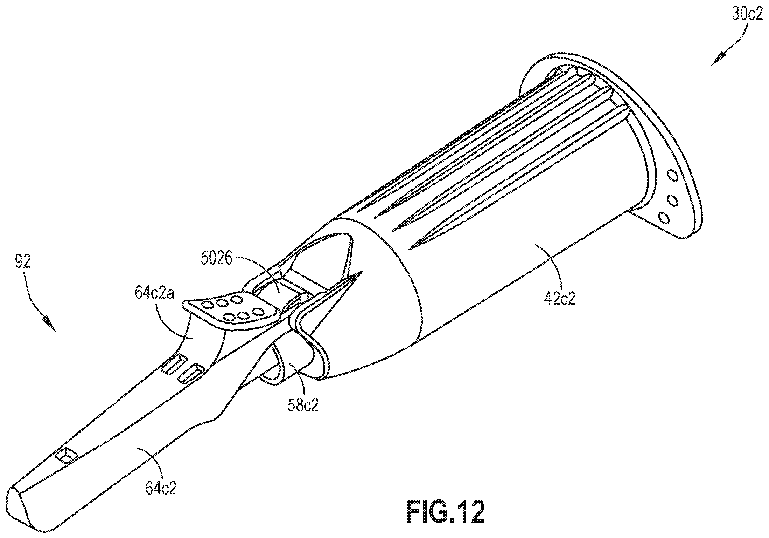

FIGS. 1 and 9-12 depict an exemplary hinged safety shield assembly embodiment of the present disclosure. A needle assembly 5000 generally includes a needle structure 32c2, associated with a hub 58c2, and a safety shield 64c2 connected to the hub 58c2 and adapted for safety shielding of the needle structure or cannula 17 after use of the device. In one embodiment, the needle assembly 5000 may incorporate features of other known needle assemblies having hinged safety shields, such as those disclosed in United States Patent Publication No. 2005/0187493.

The needle structure 32c2 may include a patient needle portion or cannula 17. Cannula 17 represents a patient end of the needle structure 32c2, and may be beveled to define a puncture tip for puncturing the skin of a patient and accessing the vasculature of the patient.

The hub 58c2 may include a front hub portion 5010 and a rear hub portion 5012 and is capable of supporting the needle structure 32c2 therethrough. In one embodiment, the cannula 17 may be integral with the front hub portion 5010, while a central lumen 5006 may be integral with the rear hub portion 5012. The front hub portion 5010 and the rear hub portion 5012 are structured to matingly engage. The front hub portion 5010 may include a protrusion 5014, such as a raised annular ring, for engaging a corresponding recess 5016 integral to the rear hub portion 5012. In another embodiment, the front hub portion 5010 and the rear hub portion 5012 may be joined together via an adhesive or weld. Once assembled, the hub 58c2 defines a flashback indicator 60c2 therein.

The hub 58c2 may further include a collar 5018 for surrounding at least a portion of the safety shield 64c2. In one embodiment, the front hub portion 5010 includes a first collar portion 5022 and the rear hub portion 5012 includes a second collar portion 5024. The first collar portion 5022 may include a generally c-shaped region 5028 for accommodating an attachment bearing 5026 of the safety shield 64c2. The attachment bearing 5026 may be integral with the safety shield 64c2. The attachment bearing 5026 may also be integral with a portion of the hub 58c2, such as the first collar portion 5022 and/or the second collar portion 5024. Alternatively, the attachment bearing 5026 may be separately provided and subsequently assembled with the safety shield 64c2 and/or the hub 58c2. The first collar portion 5022 may include a protrusion 5034 for engaging a corresponding recess 5036 integral to the second collar portion 5024. Accordingly, in one embodiment, the engagement of the front hub portion 5010 with the rear hub portion 5012 also engages the first collar portion 5022 with the second collar portion 5024. In another embodiment, the collar 5018 is positioned substantially on a top surface of the hub 58c2 to allow the safety shield 64c2 to likewise be connected to the top surface of the hub 58c2.

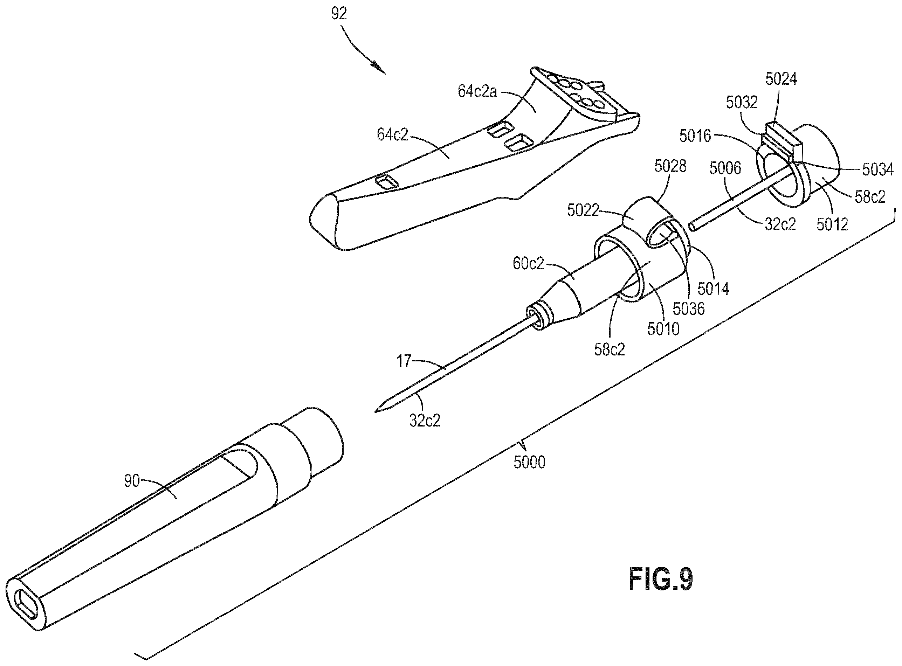

Referring to FIGS. 1 and 9, a protective cap 90 can be provided over the patient needle portion or cannula 17 prior to use, as described herein.

During use, the protective cap 90 can be removed from cannula 17 thereby exposing the cannula 17 for use. The cannula 17 can then be engaged with a patient for collecting a blood sample. After use, the safety shield 92 is used to protectively cover and shield the cannula 17 thereby preventing accidently needle stick injuries.

The needle assembly 5000 can be transitioned from a retracted position in which the cannula 17 is unshielded for the purpose of accessing a patient, to the extended position, in which the cannula 17 is safety shielded from exposure.

In some embodiments, the present disclosure provides a biological fluid collection system 10 that includes a power source 16 for a collection module 14 that receives a sample 12 and provides flow-through blood stabilization technology and a precise sample dispensing function for point-of-care and near patient testing applications. A power source of the present disclosure allows a user activated vacuum source.

In one embodiment, the power source 16 includes a spring loaded device for automatic drawing of a blood sample 12 within the collection module 14. A spring loaded power source utilizes a user activated, spring powered piston to generate a vacuum on a distal end of a collection module 14. In such an embodiment, by controlling the stiffness of and travel length of the spring, a predictable vacuum can be applied to a fluid path of the collection module 14 to generate a given flow rate of blood as it fills the collection module 14. Predictable flow rates are important for the mixing structure.

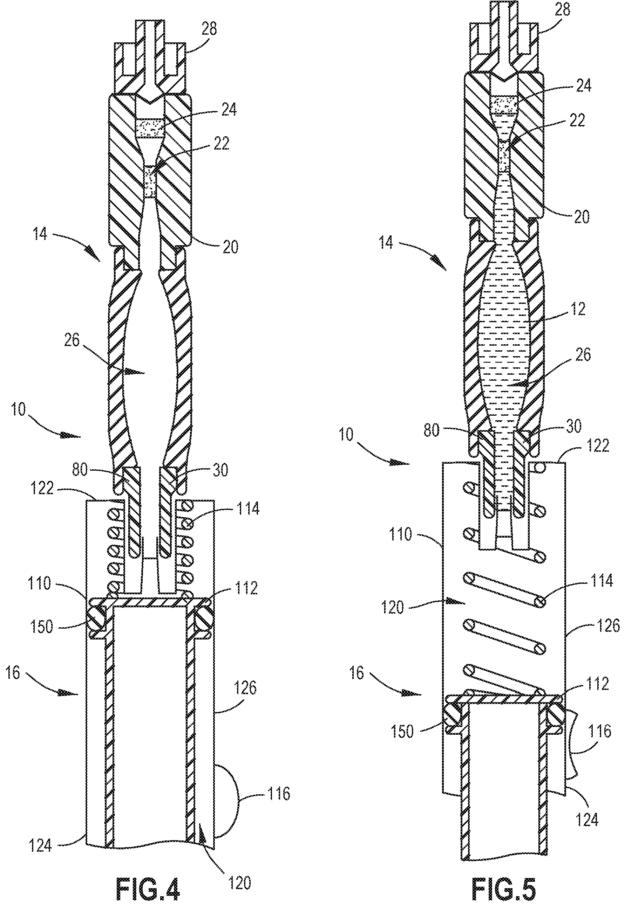

Referring to Figs. 4-5, in one exemplary embodiment, a power source 16 is removably connectable with a collection module 14 and the power source 16 creates a vacuum that draws a sample 12 within the collection chamber 26. In one embodiment, the power source 16 includes a barrel 110, a piston 112, a spring 114, an activation button 116, and a lock. In one embodiment, the piston 112 includes an O-ring 150 that provides stiction with the interior surface of a sidewall 126 of the barrel 110.

Optionally, the power source 16 may include a lock transitionable between a locked position, in which the lock locks that piston 112 in the first piston position and maintains the spring 114 in a compressed position, and an unlocked position, in which the piston 112 is unlocked and the spring 114 is permitted to drive the piston 112 to the second piston position, thereby creating a vacuum that draws the sample within the collection chamber 26. Optionally, activation of an activation member can move the lock to the unlocked position.

The barrel 110 is in communication with the collection chamber 26 of the collection module 14. The barrel 110 defines an interior 120 and includes a first end 122, a second end 124, and a sidewall 126 therebetween. The barrel 110 is removably connectable with a portion of the collection module 14. For example, in one embodiment, the barrel 110 is removably connectable with the cap 30 of the collection module 14 such that a vacuum created by the power source 16 is able to draw a sample 12 within the collection chamber 26 of the collection module 14. As discussed above, the cap 30 includes a venting plug 80 which allows air to pass therethrough and prevents the sample 12 from passing therethrough. In this manner, the vacuum created within the barrel 110 of the power source 16 is in communication with the collection chamber 26 of the collection module 14 such that a vacuum created by the power source 16 is able to draw a sample 12 within the collection chamber 26 of the collection module 14.

The piston 112 is slidably disposed within the interior 120 of the barrel 110. The piston 112 is sized relative to the interior 120 of the barrel 110 to provide sealing engagement with the sidewall 126 of the barrel 110. The piston 112 is transitionable between a first piston position (Fig. 4), in which the piston 112 is a first distance from the first end 122 of the barrel 110, and a second piston position (Fig. 5), in which the piston 112 is a second distance from the first end 122 of the barrel 110, the second distance greater than the first distance.

Referring to Figs. 4-5, the spring 114 is disposed between the first end 122 of the barrel 110 and the piston 112. In one embodiment, the activation button 116 is disposed on a portion of the barrel 110.

The power source 16 also includes a lock that is in communication with the spring 114 and the activation button 116. The lock is transitionable between a locked position, in which the lock locks the piston 112 in the first piston position (Fig. 4) and maintains the spring 114 in a compressed position, and an unlocked position, in which the piston 112 is unlocked and the spring 114 is permitted to drive the piston 112 to the second piston position (Fig. 5) thereby creating a vacuum that pulls the sample 12 within the collection chamber 26 of the collection module 14. In one embodiment, actuation of the activation button 116 moves the lock to the unlocked position.

Advantageously, a collection module and a power source of the present disclosure can be engaged with many different sources through which biological fluid, such as a blood sample 12, is passed. For example, in some embodiments, a collection module and a power source of the present disclosure can be engaged with a conventional tube holder. In other embodiments, a user activated power source of the present disclosure enables the user to connect directly to a Luer-line, e.g., IV Catheter, wingset, PICC, or similar device. In other embodiments, if the collection module and the power source are used with a HemoLuer, a user may connect the collection module and the power source to either a Luer (by removing the HemoLuer) or a conventional tube holder (using the HemoLuer as an interface). Advantageously, the system of the present disclosure also allows for direct Luer access without the use of an LLAD (Luer Line Access Device) or any other holder.

In one embodiment, a portion of the collection module 14 includes a cannula 17 for obtaining a blood sample 12 from a patient into the collection module 14. In such embodiments, the collection module 14 includes a first removable protective cap 90 and a safety shield 92. In such embodiments, the cannula 17 can be engaged with a patient and then the power source 16 used to create a vacuum that draws a sample 12 within the collection chamber 26.

In one embodiment, the blood sample 12 is pulled into the passageway 36 of the housing 20 of the collection module 14 by the draw of the vacuum created in the barrel. In one embodiment, the blood sample 12 fills the entire passageway 36 such that, as the blood sample 12 enters the collection module 14, the blood sample 12 passes through the open cell foam, e.g., the material 40, and is exposed to the anticoagulant powder available throughout the internal pore 42 structure of the open cell foam. In this manner, the sample 12 dissolves and mixes with the dry anticoagulant powder while passing through the material 40 or open cell foam. Next, the mixing chamber 22 receives the sample 12 and the sample stabilizer 24 therein and effectuates distributed mixing of the sample stabilizer 24 within the sample 12. After passing through the mixing chamber 22, the stabilized sample is directed to the collection chamber 26. The collection chamber 26 may take any suitable shape and size to store a sufficient volume of blood necessary for the desired testing, for example, 500 µl or less. In one embodiment, the cap 30 stops the collection of the blood sample 12 when the passageway 36, the mixing chamber 22, and the collection chamber 26 of the collection module 14 have been fully filled. The venting plug 80 of the cap 30 allows air to pass through the cap 30 while preventing the blood sample 12 from passing through the cap 30 into the barrel 110 of the power source 16.

In one embodiment, once sample collection is complete, the power source 16 is separated from the collection module 14. Once the collection module 14 is separated from the power source 16, the cap 30 may then be removed from the collection module 14 exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30.

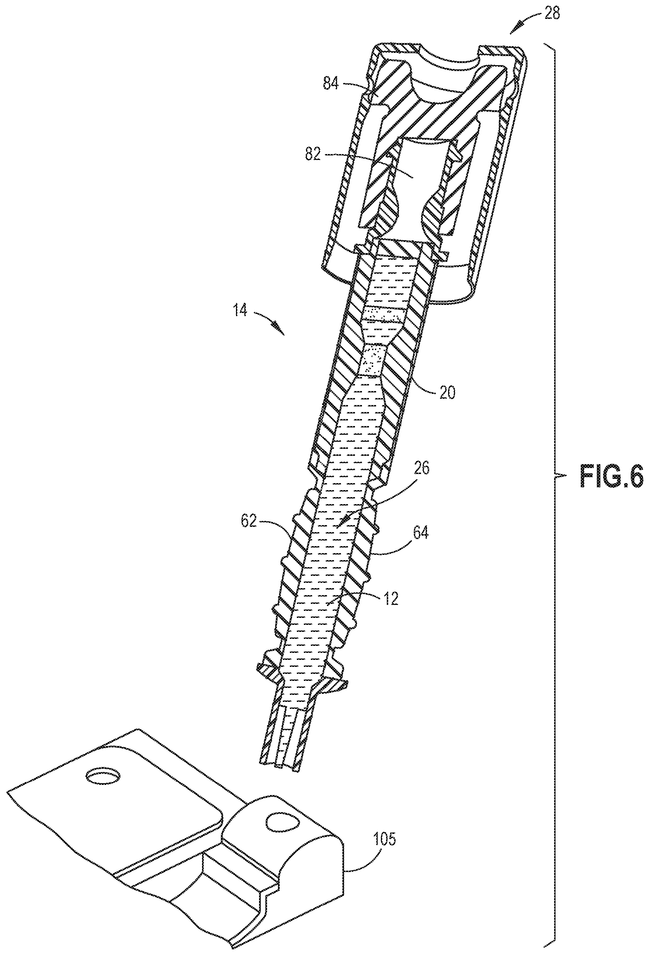

The blood sample 12 may then be dispensed from the collection module 14 by activation of the actuation portion 61. In one embodiment, the actuation portion 61 includes a first deformable portion 62 and a second deformable portion 64. For example, the first deformable portion 62 and the second deformable portion 64 are transitionable between an initial position in which the sample 12 is contained within the collection chamber 26 and a deformed position in which a portion of the sample 12 is expelled from the collection chamber 26 and the outlet port 34. The first deformable portion 62 and the second deformable portion 64 are simultaneously squeezed to transition from the initial position to the deformed position. In this manner, the blood sample 12 may be transferred to a device intended to analyze the sample, e.g., such as a point-of-care testing device 105 (Fig. 6), a cartridge tester, or a near patient testing device, while minimizing the exposure of the medical practitioner to the blood sample.

Advantageously, by having a first deformable portion 62 and a second deformable portion 64 that can be simultaneously squeezed, a collection module 14 of the present disclosure is able to dispense more sample 12 out of the collection chamber 26 and the outlet port 34. Furthermore, in one embodiment, by having a first deformable portion 62 on a first side 70 and a second deformable portion 64 on an opposite second side 72, a collection module 14 of the present disclosure has a symmetrical design and provides a smooth straight fluid path chamber that encourages fluid attachment flow characteristics.

The present disclosure provides a biological fluid collection system 10 that includes a power source 16 for a collection module 14 that receives a sample 12 and provides flow-through blood stabilization technology and a precise sample dispensing function for point-of-care and near patient testing applications. A power source of the present disclosure allows a user activated vacuum source.

A power source 16 of the present disclosure may comprise power systems in accordance with other power systems described in U.S. Patent Application Serial No. 62/658,737, filed April 17, 2018, entitled "Biological Fluid Collection System' .

As described herein, the present disclosure provides a biological fluid collection system that includes a power source for a collection module that receives a sample and provides flow-through blood stabilization technology and a precise sample dispensing function for point-of-care and near patient testing applications. A power source of the present disclosure provides a user activated vacuum source for drawing a biological fluid sample within a collection module.

A collection module of the present disclosure is able to effectuate distributed mixing of a sample stabilizer within a blood sample and dispense the stabilized sample in a controlled manner. In this manner, a biological fluid collection system of the present disclosure enables blood micro-sample management, e.g., passive mixing with a sample stabilizer and controlled dispensing, for point-of-care and near patient testing applications.

Advantageously, a biological fluid collection system of the present disclosure provides a consistent blood sample management tool for point-of-care and near patient testing applications, automatic blood draw, passive mixing technology, and controlled small sample dispensing capability to point-of-care cartridge and standard luer interfaces with near patient testing receiving ports.

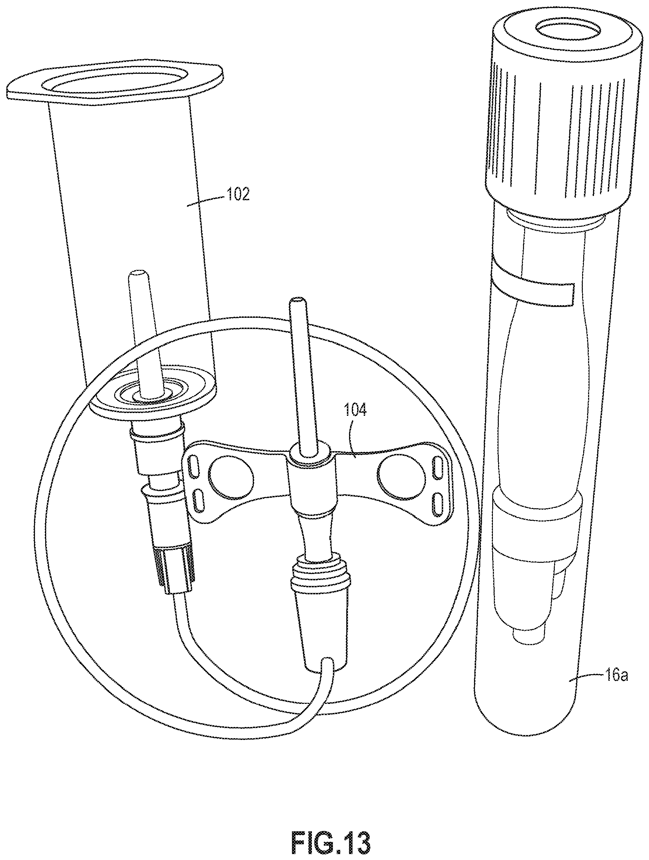

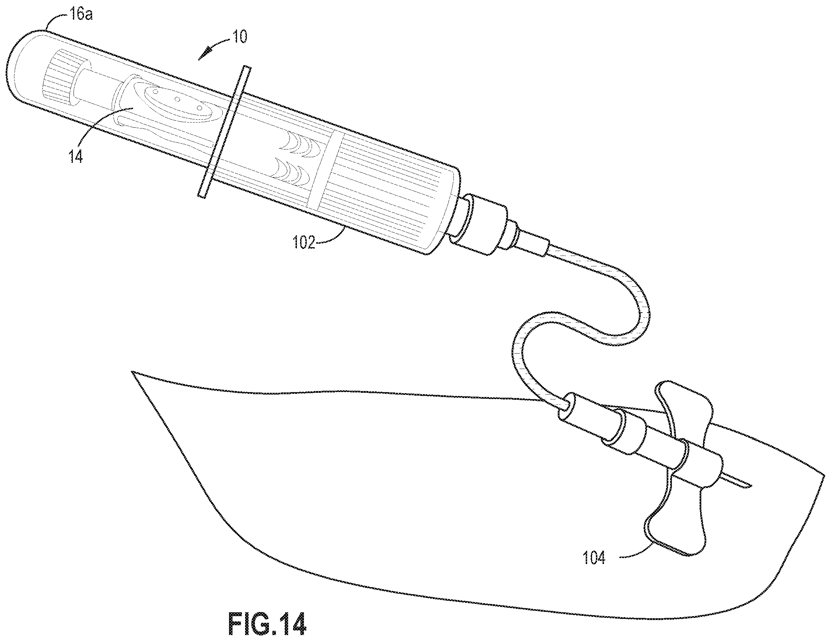

Referring to Figs. 13-14, in one embodiment, a biological fluid collection device 10 of the present disclosure is adapted to receive a biological fluid sample, such as a blood sample 12, and includes a collection module 14 and an outer housing 16a that is removably connectable to the collection module 14. In one embodiment, with the collection module 14 connected to the outer housing 16a, the collection module 14 is disposed within the outer housing 16a as shown in Figs. 13-14. The outer housing 16a may be a vacuum containing blood collection tube such as a Vacutainer^{®} blood collection tube commercially available from Becton, Dickinson and Company.

In one embodiment, the collection module 14 is disposed within the outer housing 16a and is compatible with a tube holder 102 having a cannula and a wingset 104. In use, a needle cannula of the tube holder 102 is inserted into the passageway 36 of the housing 20 of the collection module 14 through the inlet port 32, such as through the pierceable self-sealing stopper 82 of closure 28. The biological fluid collection device 10 including the combined collection module 14 and the outer housing 16a may be inserted into a conventional tube holder 102 having a cannula through which biological fluid, such as a blood sample 12, is passed.

The blood sample 12 is pulled into the passageway 36 of the housing 20 of the collection module 14 from the conventional tube holder 102 by the draw of the vacuum contained in the outer housing 16a. In one embodiment, the blood sample 12 fills the entire passageway 36 such that, as the blood sample 12 enters the collection module 14, the blood sample 12 passes through the open cell foam, e.g., the material 40, and is exposed to the anticoagulant powder 44 available throughout the internal pore 42 structure of the open cell foam. In this manner, the sample 12 dissolves and mixes with the dry anticoagulant powder 44 while passing through the material 40 or open cell foam. Next, the mixing chamber 22 receives the sample 12 and the sample stabilizer 24 therein and effectuates distributed mixing of the sample stabilizer 24 within the sample 12. After passing through the mixing chamber 22, the stabilized sample is directed to the collection chamber 26. The collection chamber 26 may take any suitable shape and size to store a sufficient volume of blood necessary for the desired testing, for example 500 µl or less. In one embodiment, the cap 30 stops the collection of the blood sample 12 when the passageway 36, the mixing chamber 22, and the collection chamber 26 of the collection module 14 has been fully filled. The venting plug 80 of the cap 30 allows air to pass through the cap 30 while preventing the blood sample 12 from passing through the cap 30 into the outer housing 16.

In one embodiment, once sample collection is complete, the outer housing 16a including the collection module 14 is separated from the tube holder 102, and then the outer housing 16a is separated from the collection module 14 by removing the closure 28, which is still attached to the collection module 14, from the outer housing 16a. Removal of the closure 28 may be accomplished by the user grasping both the outer shield 84 of the closure 28 and the outer housing 16a and pulling or twisting them in opposite directions.

Once the collection module 14 is separated from the outer housing 16a, the cap 30 may then be removed from the collection module 14 exposing the outlet port 34 of the housing 20 of the collection module 14. Removal may be accomplished by the user grasping an exterior portion of the cap 30 and pulling the cap 30 from the housing 20. The blood sample 12 is held within the passageway 36 of the housing 20, e.g., the collection chamber 26, by capillary action after removal of the cap 30. In one embodiment, alternatively, removal of the cap 30 may occur upon removal of the collection module 14 from the outer housing 16. In this configuration, the cap 30 is restrained within the outer housing 16. In one embodiment, the cap 30 may be engaged with the outer housing 16 so that the outer housing 16 and the cap 30 are removed in a single step.

The blood sample 12 may then be dispensed from the collection module 14 by activation of the first deformable portion 62 and the second deformable portion 64. For example, the first deformable portion 62 and the second deformable portion 64 are transitionable between an initial position in which the sample 12 is contained within the collection chamber 26 and a deformed position in which a portion of the sample 12 is expelled from the collection chamber 26 and the outlet port 34. The first deformable portion 62 and the second deformable portion 64 are simultaneously squeezed to transition from the initial position to the deformed position. In this manner, the blood sample 12 may be transferred to a device intended to analyze the sample, e.g., such as a point-of-care testing device 105 (Fig. 6), a cartridge tester, or a near patient testing device, while minimizing the exposure of the medical practitioner to the blood sample.

The present disclosure includes blood collection devices for collecting of arterial blood samples using a radial stick technique. The present disclosure streamlines and reduces the number of workflow steps which is very important in an Arterial Blood Gas (ABG) collection procedure. The proposed device includes automatic anticoagulant mixing and integrated venting for expelling air bubbles during collection.

The existing ABG syringes typically uses conventional hypodermic needles with a safety shield that need to be snapped or slide over the needle after the blood collection. Such safety guards are often in line of sight during the blood collection procedure therefore obscuring the physician's view during this delicate procedure. The conventional ABG syringes often also have a separate vent cap that requires the needle to be removed before the cap is attached to the syringe to expel trapped air bubbles from the collected sample. Anticoagulant is typically loaded inside the ABG syringe requiring a user to roll or shake a collected sample to ensure thorough mixing with anticoagulant. A device of the present disclosure has the following advantages over conventional ABG collection kits: (1) ergonomic design with designated touch points; (2) thin wall needle technology (BD Ultratouch) that allows a small gage needle while still maintaining high flow rate resulting in fast fill time and shorter patient exposure to potentially painful procedures; (3) push-button safety shield that could be singlehanded activated by simple push of the button after the collection while not obscuring a user's view during the procedure; (4) integrated air venting/venting cap capability which allows removal of trapped air bubbles by simple expelling during fill or after collection; and (5) utilizes a safety sleeve with guard and push button that prevents accidental safety shield activation during the device transport.

In another concept of the present disclosure, referring to Figs. 15-27, a fluid collection assembly 10b generally includes a needle assembly 11b, a first needle shield 60b, a tube holder 13b and a fluid collection cartridge 20b. According to one embodiment, the fluid collection assembly 10b can comprise an arterial blood collection assembly. While described herein in terms of an arterial blood collection cartridge 20b intended for use with a needle assembly 11b, the cartridge 20b of the present disclosure may be used with or may incorporate other medical devices, such as another medical device assembly that includes a piercing element or allows for attachment to a catheter or arterial lines.

In an exemplary embodiment, the primary components of the fluid collection cartridge 20b include a plunger assembly 30b having a removable plunger rod 31b and a stopper 32b in slidable communication with a tubular member 21b and a closure 40b.

Referring to Figs. 15-18, in one exemplary embodiment, a fluid collection cartridge 20b includes an elongated, hollow, cylindrically-shaped tubular member 21b having a proximal end 23b, an open distal end 22b, and a sidewall 25b extending between the proximal end 23b and the distal end 22b defining an internal chamber 26b having an internal reservoir 28b. The sidewall 25b of tube 21b defines an internal surface 27b for slidably receiving a plunger assembly 30b. An annular flange 24b is provided at the proximal end 23b of the tubular member 21b and extends from the internal surface 27b into chamber 26b partially closing the proximal end 23b of the tubular member 21b.

Tubular member 21b may be made of one or more than one of the following representative materials: polypropylene, polyethylene, polyethyleneterephthalate (PET), polystyrene, polycarbonate, cellulosics, glass products, or combinations thereof. More expensive plastics such as polytetrafluoroethylene and other fluorinated polymers may also be used. In addition to the materials mentioned above, examples of other suitable materials include polyolefins, polyamides, polyesters, silicones, polyurethanes, epoxies, acrylics, polyacrylates, polysulfones, polymethacrylates, PEEK, polyimide and fluoropolymers such as PTFE Teflon^{®}, FEP Teflon^{®}, Tefzel^{®}, poly(vinylidene fluoride), PVDF, and perfluoroalkoxy resins. One exemplary glass product is PYREX^{®} (available from Corning Glass, Corning, New York). Ceramic collection devices can be used according to embodiments of the invention. Cellulosic products such as paper and reinforced paper containers can also be used to form collection devices according to the present disclosure.

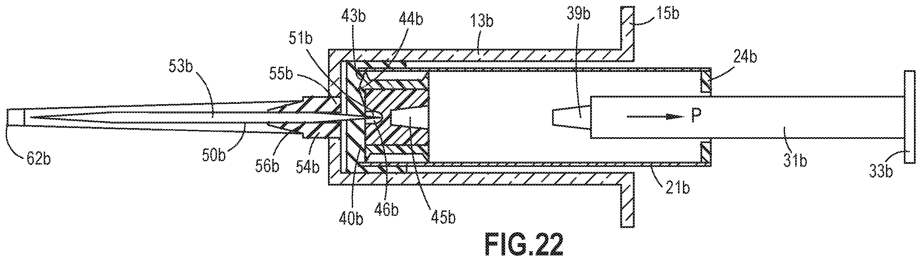

Referring to Figs. 15-18, in one exemplary embodiment, the fluid collection cartridge 20b also includes a plunger assembly 30b slidably received within the chamber 26b defined by sidewall 25b of the tube 21b. The plunger assembly 30b includes a stopper 32b and a removable plunger rod 31b. The stopper 32b is slidably positioned in fluid tight engagement with internal surface 27b, and is able to slide distally and proximally along a longitudinal axis 29b. The stopper 32b and plunger rod 31b are removably associated with one another by an interengaging arrangement 90b. The interengaging arrangement 90b is configured to enable the plunger rod 31b to apply a distally directed force to the stopper 32b and to enable removal of the plunger rod 31b from the stopper 32b and from the tubular member 21b upon the application of a proximally directed force, as shown by "P" in FIG. 22. In other words, the stopper 32b and plunger rod 31b are not mechanically secured to each other but merely are arranged in a removable contacting arrangement which only allows plunger rod 31b to exert a force in a distal direction on stopper 32b. A proximal end of the removable plunger rod 31b may include a thumb flange 33b that a user may use to exert a force to push the entire plunger assembly 30b distally, or pull to remove the plunger rod 31b from tubular member 21b.

Referring to Figs. 15-18, in one exemplary embodiment, stopper 32b includes a distal face 34b and a proximal face 35b. The diameter of stopper 32b is approximately equal to or only slightly smaller than the internal diameter 'a' of the tube 21b but is greater than the internal diameter 'b' of annular flange 24b. Stopper 32b is in slidable contact with internal surface 27b of tube 21b and provides a fluid-tight seal between the plunger assembly 30b and the internal surface 27b of the tube 21b so that a sample can be held within the internal reservoir 28b formed within the chamber 26b between distal end 22b of tube 21b and distal face 34b of stopper 32b, thereby preventing the sample from leaking from the proximal end 23b of tube 21b.

Stopper 32b is a low resistance stopper and as such is designed to have a relatively lower frictional resistance to movement inside of tube 21b when compared to similar components in prior art arterial blood gas syringes such that the presence of fluid pressure, such as arterial blood pressure, within internal reservoir 28b will cause the stopper 32b to slide/travel in a proximal direction toward the proximal end 23b of tube 21b until the proximal face 35b of the stopper 32b contacts annular flange 24b thereby limiting the proximal movement of stopper 32b. The frictional resistance of a stopper can be lowered by either a combination of stopper sealing profile design and/or component material selection. The first 36b and second 37b sealing rings extend around the outer circumferential surface of stopper 32b near the distal face 34b and proximal face 35b, respectively, to create a primary and secondary seal with internal surface 27b of tube 21b. This stopper sealing profile design lowers the amount of contact between stopper 32b and internal surface 27b thereby reducing the frictional resistance to movement of stopper 32b when compared to a stopper sealing profile in which the entire outer circumferential surface is in contact with internal surface 27b. Alternately or in combination with the stopper sealing profile design, stopper 32b is preferably made of an elastomeric material such as natural rubber, synthetic rubber, thermoplastic elastomers, and combinations thereof which are formulated or synthesized to be self-lubricating or have relatively lower frictional resistance. Stopper 32b may also be made from a combination of elastomers which include a harder inner rubber core and a soft self-lubricating polymeric material outer layer. A self-lubricating polymeric material has a lubricant such as silicone oil incorporated into the polymeric material, an example of which is Epilor.

Prior to use, plunger rod 31b contacts the proximal face 35b of stopper 32b in such a manner that plunger rod 31b can only impart a force applied in the distal direction. The interengaging arrangement 90b can include a male member, such as a conical finger 39b, extending from a distal end of plunger rod 31b which is configured to mate with a corresponding female member, such as a conical recess 45b, in the proximal face 35b of stopper 32b. It can be appreciated that the conical finger 39b and conical recess 45b illustrate one example of an interengaging arrangement 90b and that other interengaging arrangements can be used to removably connect the plunger rod 31b with the stopper 32b. For example, the interengaging arrangement 90b can be designed such that the distal end of the plunger rod 31b includes a female member configured to mate with a corresponding male member extending from the proximal face 35b of the stopper 32b.

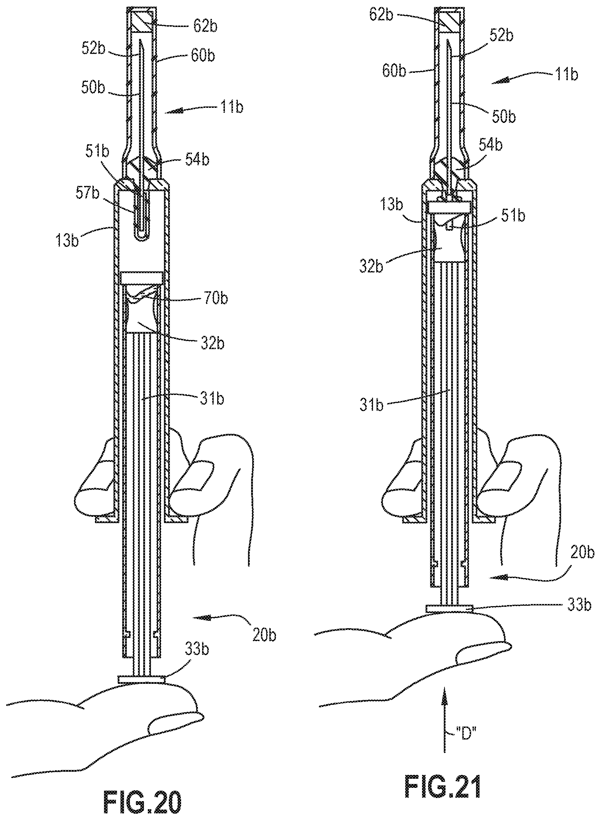

Referring to Figs. 20-22, application of a force, such as by a user, to the thumb flange 33b causes plunger rod 31b to transmit the applied force to move stopper 32b in a distal direction to force fluids out of the blood collection cartridge should a fluid passageway be present in the distal end 22b of tube 21b. However, pulling plunger rod 31b in a proximal direction, as shown by "P" in FIG. 22, will impart no force on stopper 32b. Conical finger 39b will simply retract out of contact with recess 45b, thus stopper 32b will remain stationary in tube 21b while plunger rod 31b is removed from tubular member 21b.

Plunger rod 31b is desirably constructed of a suitable polymeric material, and may be manufactured by injection molding with a suitable polymer material known in the art. It is within the purview of the present invention to include plunger rods and stoppers which are separately formed or integrally formed of the same material or different materials such as in two-color molding, or separately formed of the same or different materials and joined together by mechanical means, adhesives, ultrasonic welding, heat sealing, or other suitable means.

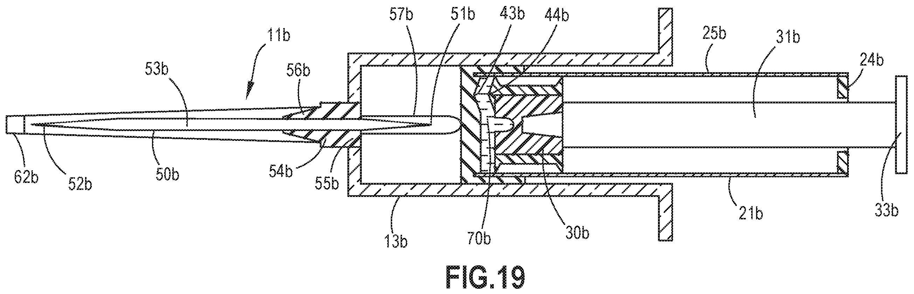

Referring to Figs. 15-18, in one exemplary embodiment, a pierceable closure 40b is associated with the open distal end 22b of the tubular member 21b. The closure 40b is configured to cooperate with the sidewall 25b of the tubular member 21b to sealingly close the open distal end 22b to form a liquid impermeable seal to contain the fluid sample. The closure 40b includes an external end 41b and an internal end 42b structured to be at least partially received within the tubular member 21b. Portions of the closure 40b adjacent the open distal end 22b of the tube 21b define a maximum outer diameter which exceeds the inside diameter 'a' of the tube 21b. The inherent resiliency of closure 40b can ensure a sealing engagement with the internal surface 27b of the wall 25b of the tube 21b. Portions of the closure 40b extending downwardly from the internal end 42b may taper from a minor diameter which is approximately equal to, or slightly less than, the inside diameter 'a' of the tube 21b, to a major diameter that is greater than the inside diameter 'a' of the tube 21b adjacent the distal end 22b. Thus, the internal end 42b of the closure 40b may be urged into a portion of the tube 21b adjacent the distal open end 22b. Closure 40b is such that it can be pierced by a needle 50b or other cannula to introduce a biological sample into tubular member 21b. According to one embodiment, closure 40b is resealable. The closure 40b can also be formed to define a cavity 43b, as shown in FIG. 19, extending into the internal end 42b. The cavity 43b may be sized to receive at least a corresponding mixing fin 44b extending distally from the distal face 34b of stopper 32b. Alternatively, a plurality of cavities and corresponding mixing fins may be present. Suitable materials for closure 40b include, for example, elastomers such as silicone rubber, natural rubber, styrene butadiene rubber, ethylene-propylene copolymers and polychloroprene, thermoplastic elastomers, and the like.

According to an embodiment, the fluid collection cartridge 20b may contain additional additives as required for particular testing procedures, such as anticoagulants, clotting agents, stabilization additives, and the like, as illustrated as 70b in FIG. 19. Such additives may be sprayed onto the internal surface 27b of the tube 21b or located within the internal reservoir 28b. The anticoagulants may include hirudins, hirudin derivatives, chelating agents, or chelating agent derivatives. Specific anticoagulants include citrate, ethylenediaminetetraacetic acid (EDTA), heparin, CPAD, CTAD, CPDA-1, CP2D, potassium oxalate, sodium fluoride, or ACD. The anticoagulant can be used in a liquid form to improve the incorporation, hence, effectiveness of the anticoagulant upon collection of arterial blood. The liquid form can be an emulsion, solution, or dispersion of the anticoagulant in a suitable carrier. Other known arterial blood sample collection methods use an arterial blood gas syringe preloaded upon manufacture with a solid form of anticoagulant such as heparin powder within the syringe barrel in order to maximize the shelf life of the syringe. The use of a solid form of anticoagulant can cause a reduction in the effectiveness of the anticoagulant as the incorporation of powdered heparin into the blood sample is difficult due to lack of agitation during the arterial blood collection process.

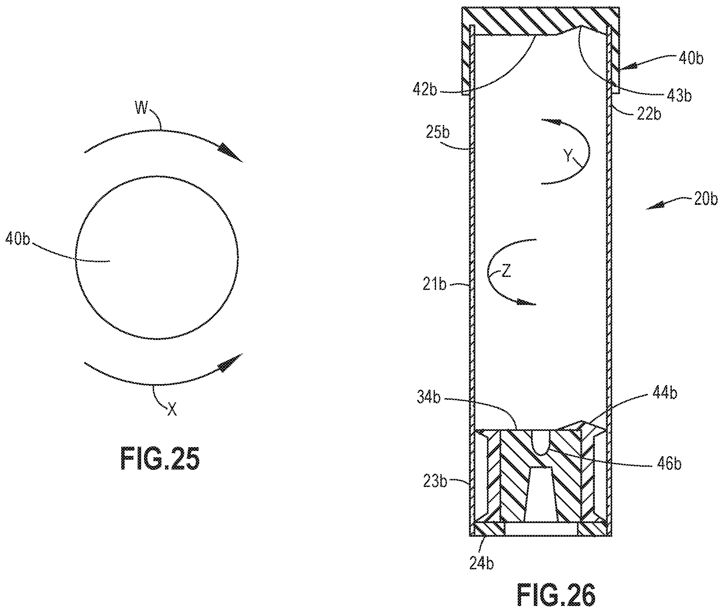

The combination of a cavity 43b in the internal end 42b of closure 40b and a mixing fin 44b extending from distal face 34b of stopper 32b provides asymmetric surfaces at each end of the fluid reservoir 28b. Referring to Figs. 25-26, when the cartridge 20b is rolled by rotating the cartridge about longitudinal axis in the direction of arrows w and x, the cavity 43b and mixing fin 44b create vortices that promote thorough mixing of the contents of the fluid reservoir 28b. This is particularly useful in instances where there is no air (headspace) in the fluid reservoir of a collection vessel. Tipping such devices end-over-end does little to mix the contents, especially if the components are similar in density. Vessels with a cylindrical internal fluid reservoir such as vials, insulin pen cartridges, and syringes typically have flat internal surfaces in the top and bottom of the fluid reservoir. Therefore, little turbulence is created when these vessels are rolled.

Referring to Figs. 15-22, an embodiment of a fluid collection system is shown including a needle assembly 11b, and a holder 13b. The needle assembly 11b includes a needle cannula 50b having a pointed proximal end 51b, a pointed distal end 52b, and a lumen 53b extending between the proximal end 51b and distal end 52b. The needle assembly 11b further includes a hub 54b having a proximal end 55b, a distal end 56b, and a passage extending between the proximal end 55b and distal end 56b. Portions of the needle cannula 50b extending between the proximal end 51b and distal end 52b are mounted securely in the passage of the hub 54b. Thus, the pointed proximal end 51b of the needle cannula 50b projects proximally beyond the hub 54b and the pointed distal end of the needle cannula projects distally beyond the hub 54b. External surface regions of the hub 54b near the proximal end 55b of the hub 54b may be formed with mounting structures, such as an array of external threads, at least one annular groove, or at least one annular rib. The mounting structure enables the needle hub 54b to be secured to a holder 13b that is configured to slidably receive a blood collection cartridge 20b according to an embodiment of the present disclosure. The needle assembly 11b may further include a multiple sample sleeve 57b, as shown in FIG. 19, mounted over the proximal portion of the needle cannula 50b and secured to the proximal end 55b of the hub 54b. The proximal portions of the needle cannula 50b and the multiple sample sleeve 57b project into the holder 13b when the hub 54b of the needle assembly 11b is mounted to the holder 13b.

Referring to Figs. 15-22, in an exemplary embodiment, needle assembly 11b includes a first needle shield 60b and a second needle shield 61b. First needle shield 60b covers distal end 52b of cannula 50b while second needle shield 61b covers the proximal end 51b of cannula 50b. First needle shield 60b includes an indicator tip 62b at the distal end that is activated by the presence of a fluid treatment material 70b, such as a liquid anticoagulant.

Assembly of the fluid collection cartridge 20b is accomplished by slidably inserting stopper 32b within chamber 26b through distal end 22b of tubular member 21b. Fluid treatment material 70b, such as liquid anticoagulant heparin, is then added to fluid reservoir 28b before distal end 22b is sealed by the insertion of closure 40b. Plunger rod 31b is then inserted through annular flange 24b at proximal end 23b of tube 21b until conical finger 39b mates with recess 45b. The assembly can then be packaged for later use.

In a method which is not according to the present invention, second needle shield 61b is removed from needle assembly 11b and holder 13b connected for fluid collection, such as for arterial blood collection. A fluid collection cartridge 20b in accordance with an embodiment of the invention, such as a blood collection cartridge, is then inserted into the proximal end of holder 13b as shown in FIGS. 19-21 until pointed proximal end 51b of needle 50b penetrates closure 40b and lumen 53b is in fluid communication with the fluid treatment material 70b, such as liquid heparin anticoagulant, located in fluid reservoir 28b.

A user then grips the holder 13b, anchors fingers about an outwardly extending annular flange 15b on the holder 13b, and presses down upon thumb flange 33b with sufficient force in a distal direction "D", as shown in FIG. 21, until the distal face of stopper 32b contacts internal end 42b of closure 40b and the internal mixing fin 44b mates with recess 45b and proximal end 51b of cannula 50b is accommodated in cavity 43b of stopper 32b, as shown in FIG. 22. This action causes the fluid treatment material 70b within the fluid collection cartridge 20b to flow from fluid reservoir 28b via proximal end 51b into lumen 53b of cannula 50b and into the first needle shield 60b. The indicator tip 62b will activate and change color upon contact with the excess fluid treatment material as it is expelled from distal end 52b of needle 50b, to give the user a visual confirmation that any dead space within fluid reservoir 28b or lumen 53b of the needle 50b is primed with the fluid treatment material 70b. The plunger rod 31b can then be separated from the stopper 32b by pulling plunger rod 31b in a proximal direction (indicated by arrow P) as shown in FIG. 22. The residual volume (10-20 µl) of fluid treatment material 70b which is present in the dead space should be at a concentration so as to provide sufficient treatment, such as providing sufficient anticoagulant function to prevent clotting of an arterial blood sample upon collection.

The purpose of priming assembly 10b with a fluid treatment material is to remove any atmospheric air, so that the partial pressure of the oxygen, such as in an arterial blood sample, will not be affected by the atmospheric air. The assembly 10b should preferably have low dead space to keep the residual volume of the fluid treatment material low in order to minimize the dilution effect of the fluid treatment material on the fluid sample.

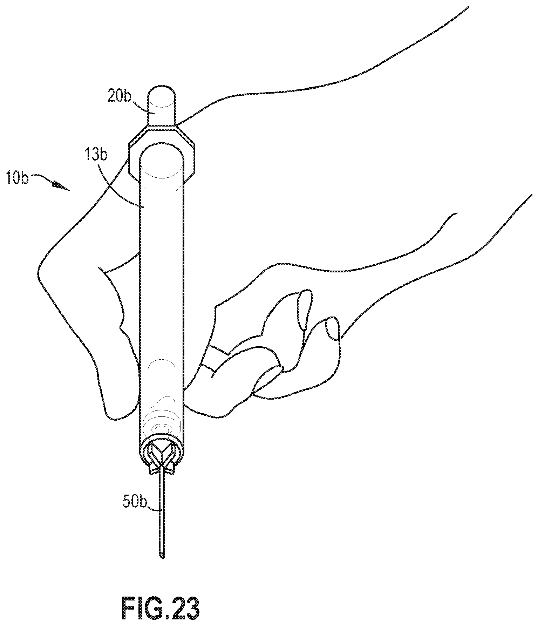

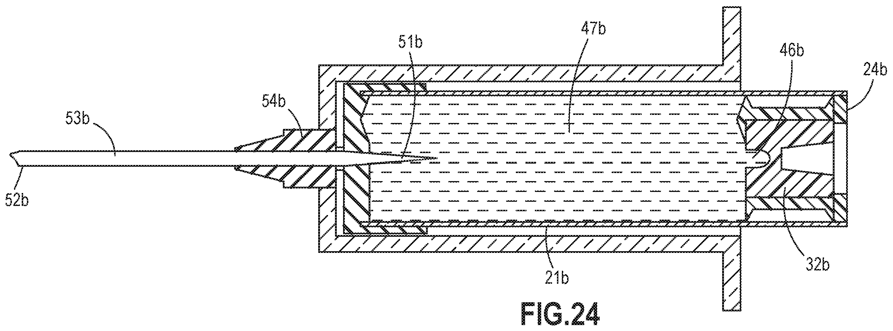

A method of fluid collection which is not according to the present invention enables a single-handed technique similar to current clinical practice in the fluid collection process or an arterial blood collection process using a low resistance rubber stopper that is moved by the arterial pressure. First needle shield 60b is removed from needle assembly 11b. The user grips assembly 10b as shown in FIG. 23 with one hand and inserts distal end 52b into a fluid source, such as a patient's artery. When removing arterial blood, the blood at arterial pressure (which is greater than normal atmospheric or ambient pressure) will then flow through lumen 53b of cannula 50b into the fluid reservoir 28b and forces stopper 32b to slide in a proximal direction until the stopper proximal face 35b contacts annular flange 24b thereby defining the completion of the collection volume of the blood sample as shown in FIG. 24. The sliding motion of the rubber stopper 32b allows the fluid treatment material 70b and the collected arterial blood to mix, as shown at 47b, during the collection process.

Fluid or blood collection cartridge 20b is then removed from the multi-sample needle assembly 11b and holder 13b. The distal end 52b can then be removed from the fluid source or artery. The detached cartridge 20b may then be rolled between the user's palms in a plane perpendicular to longitudinal axis 29b in order to further mix the fluid sample with a fluid treatment material 70b, such as heparin, as shown in FIGS. 25-26. The asymmetrical finned surfaces of the internal end 42b of closure 40b and the distal face 34b of stopper 32b create a vortex when the cartridge 20b is rolled in the directions as indicated by arrows w, x, y, and z. The fluid collection cartridge 20b containing the fluid sample is now ready for transportation to the laboratory such as for arterial blood gas analysis.

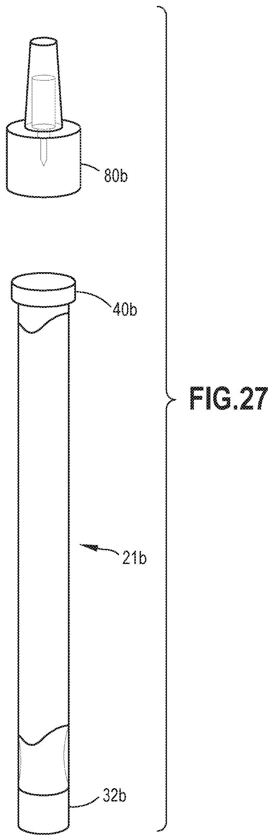

According to one embodiment, a luer adapter 80b as shown in FIG. 27 may then be inserted through closure 40b of the cartridge 20b to provide the cartridge with an interface connection that is compatible with a blood gas analyzer. A range of different luer adapters can be provided to allow the fluid collection cartridge 20b to connect to all different types of testing equipment and/or all different types of blood gas analyzer interfaces. The luer adapter 80b may also be supplied with a luer tip cap (not shown) to seal the fluid collection cartridge 20b when the luer adapter 80b is connected.

Referring to Figs. 15 and 28-30, in one embodiment, the device may also include a shieldable needle device 12. For example, referring to Figs. 15 and 28-30, fluid collection assembly 10b, 10c may include a shieldable needle device 12c.

In one exemplary embodiment, the shieldable needle device 12c may include a flexible tube 14c extending from needle device 12c, a fixture 16c mounted to flexible tube 14c, a needle cannula 20c, a hub 30c, and an outer shield 50c. In some embodiments, fixture 16c is connectable to a receptacle (not shown) for use in blood collection procedures, as is known in the art.

In one embodiment, needle cannula 20c includes a proximal end and an opposing distal end, with lumen 26c extending through needle cannula 20c from proximal end to distal end. Distal end of needle cannula 20c is beveled to define a sharp puncture tip 28c, such as an intravenous puncture tip. Puncture tip 28c is provided for insertion into a patient's blood vessel, such as a vein, and is therefore designed to provide ease of insertion and minimal discomfort during venipuncture. A removable protective cover (not shown) may be positioned over distal end of needle cannula 20c for protection from puncture tip 28c prior to use of blood collection set 10c.

Shieldable needle device 12c further includes hub 30c. Hub 30c is a unitary structure, desirably molded from a thermoplastic material. Needle cannula 20c is positioned within and is supported by an internal passageway of hub 30c, with distal end of needle cannula 20c extending from distal end of hub 30c. Desirably, needle cannula 20c and hub 30c are separate parts which are fixedly attached and secured through an appropriate medical grade adhesive or the like. Proximal end of hub 30c is adapted for connection with a flexible tube 14c of blood collection set 10c. Hub 30c further includes a first tab 40c extending outwardly from an outer surface at a location adjacent proximal end of hub 30c. In this manner, flexible tab 40c is accessible to a user's finger when shieldable needle device 12c is assembled with tube 14c in blood collection set 10c.

Shieldable needle device 12c further includes hollow outer shield 50c. Outer shield 50c further includes a second tab 62c extending outwardly from a top portion of housing 50c. Second tab 62c includes a ramped surface having protrusions thereon for providing frictional engagement with a user's thumb.

Outer shield 50c is movable between a retracted position in which first tab 40c is exposed from proximal end of outer shield 50c and puncture tip 28c is exposed from distal end of outer shield 50c, and an extended position in which puncture tip 28c and distal end of needle cannula 20c are covered by outer shield 50c.

First tab 40c and second tab 62c are configured such that opposing forces applied against first tab 40c and second tab 62c cause outer shield 50c to move toward distal end of needle cannula 20c in a direction of arrow 100c from the retracted position to the extended position. Protrusions on first tab 40c and second tab 62c, respectively, provide frictional engagement with the user's finger and thumb, respectively, to facilitate moving outer shield 50c from the retracted position to the extended position.

Outer shield 50c may further include a pair of stabilizers in the form of wings 68c extending laterally from outer shield 50c at opposing sides thereof, providing blood collection set 10c as a butterfly-type wing assembly. Wings 68c assist in positioning and placing shieldable needle device 12c and blood collection set 10c during a blood collection procedure and are adapted to lie flat against the surface of a patient's skin during the blood collection procedure. As such, wings 68c may be constructed of a flexible material such that at least one, and desirably both, of wings 68c can be bent toward each other and brought together between the fingers of the user to assist in positioning and placing shieldable needle device 12c during venipuncture.

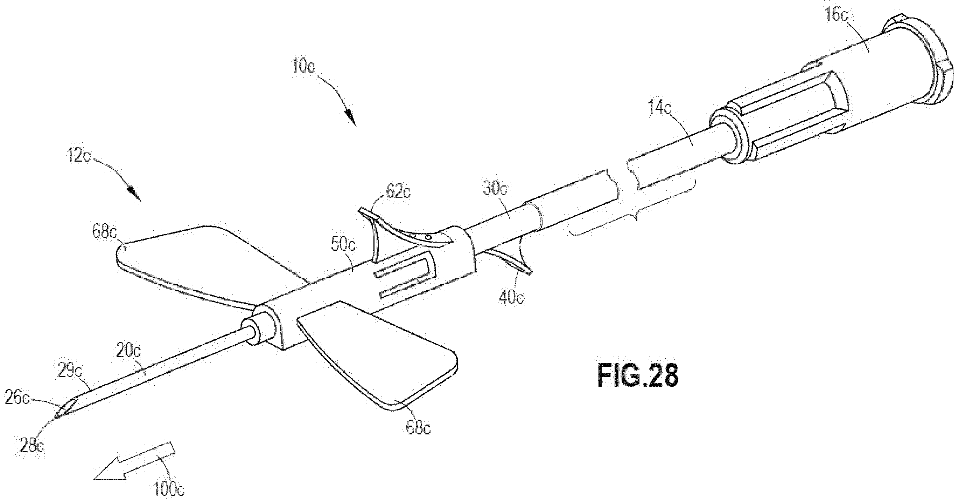

Shieldable needle device 12c can be packaged substantially in the condition shown in FIGS. 15 and 28. In particular, blood collection set 10c is provided with needle device 12c assembled and including flexible tube 14c extending from needle device 12c and connected to fixture 16c. Prior to use, blood collection set 10c is removed from its package. After removing blood collection set 10c from its package, it can be assembled with other appropriate medical equipment for use. Fixture 16c then may be connected to an appropriate receptacle, such as a non-patient needle assembly and a needle holder, for providing fluid communication with lumen 26c through needle cannula 20c.

To prepare for use of blood collection set 10c, the user grasps blood collection set 10c at shieldable needle device 12c and removes the protective cover (not shown) to expose puncture tip 28c of needle cannula 20c. The medical practitioner can then urge puncture tip 28c at distal end of needle cannula 20c into a targeted blood vessel of a patient. During such positioning, at least one of wings 68c can be bent inwardly toward the other with the user's fingers to facilitate positioning and placing of shieldable needle device 12c. Upon completion of the procedure, such as when all desired samples have been drawn, needle cannula 20c is withdrawn from the patient. After removal of needle cannula 20c from the patient, activation of the safety feature of shieldable needle device 12c is accomplished.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A fluid collection system (10) comprising:
a fluid collection cartridge (20b) comprising:
a tubular member (21b) having a proximal end (23b), an open distal end (22b), and sidewall (25b) extending between the proximal end and the distal end defining an internal chamber (26b) having an internal reservoir (28b);
a pierceable closure (40b) associated with the open distal end of the tubular member (21b), the closure configured to cooperate with the sidewall (25b) of the tubular member (21b) to sealingly close said open distal end (22b); and
a plunger rod assembly (30b) including a stopper (32b) and a plunger rod (31b) removably associated with one another by an interengaging arrangement (90b), wherein said interengaging arrangement (90b) is configured to enable the plunger rod (31b) to apply a distally directed force to the stopper (32b) and to enable removal of the plunger rod (31b) from the stopper (32b) and from the tubular member (21b) upon the application of a proximally directed force;
a needle assembly (11b) connected to the fluid collection cartridge (20b), the needle assembly (11b) comprising:
a needle cannula (50b) having a pointed proximal end (51b), a pointed distal end (52b), and a lumen (53b) extending between the proximal end (51b) and the distal end (52b);
a hub (54b) within which the needle cannula (50b) is mounted such that the pointed proximal end (51b) of the needle cannula (50b) projects proximally beyond the hub (54b) and the pointed distal end (52b) of the needle cannula (50b) projects distally beyond the hub (54b); and
a holder (13b) to which the hub (54b) is secured, with the holder (13b) configured to slidably receive the fluid collection cartridge (20b); **characterised in that** the fluid collection system further comprises
a shieldable needle device (12c) connectable to the needle assembly (11b), comprising:
a hub (30c);
a needle cannula (20c) positioned within and supported by an internal passageway of the hub (30c), the needle cannula (20c) distal from the hub (30c);
a flexible tube (14c) connectable with the hub (30c) to extend proximally therefrom;
a fixture (16c) mounted to the flexible tube (14c), on a side thereof opposite the hub (30c); and
an outer shield (50c) movable between a retracted position in which a puncture tip (28c) of the needle cannula (20c) is exposed from a distal end of the outer shield (50c), and an extended position in which the puncture tip (28c) and is covered by the outer shield (50c);
wherein
the hub (30c) further includes a first tab (40c) extending outwardly from an outer surface thereof at a location adjacent a proximal end of the hub (30c), and wherein the outer shield (50c) includes a second tab (62c) extending outwardly therefrom, with the first tab (40c) and the second tab (62c) configured such that opposing forces applied against the first tab (40c) and the second tab (62c) cause the outer shield (50c) to move toward a distal end of needle cannula (20c), from the retracted position to the extended position.

2. The fluid collection system of claim 1, wherein the stopper (32b) is in slidable contact with an internal surface (27b) of the sidewall (25b) of the tubular member (21b) and provides a fluid-tight seal therebetween, so that a sample can be held within the internal reservoir (28b), the internal reservoir (28b) formed within the chamber (26b) between the distal end (22b) of the tubular member (21b) and a distal face (34b) of the stopper (32b).

3. The fluid collection system of claim 1 or claim 2, wherein the stopper (32b) is a low resistance stopper, such that the presence of fluid pressure within the internal reservoir (28b) causes the stopper (32b) to slide in a proximal direction toward the proximal end (23b) of the tubular member (21b) until a proximal face (35b) of the stopper (32b) contacts an annular flange (24b) at the proximal end of the tublular member (21b).

4. The fluid collection system of claim 3, wherein the stopper (32b) is a self-lubricating stopper.

5. The fluid collection system of any of claims 1-4, wherein the pierceable closure (40b) is configured to be pierced by a needle (50b) to introduce a biological sample into the tubular member (21b), with the pierceable closure (40b) configured to reseal after removal of the needle (50b).

6. The fluid collection system of any of claims 1-5, wherein the pierceable closure (40b) defines a cavity (43b) extending into an internal end (42b) of the pierceable closure (40b), and wherein a distal face (34b) of the stopper (32b) comprises a mixing fin (44b) extending distally therefrom that is sized to be received within the cavity (43b).

7. The fluid collection system of claim 6, wherein the cavity (43b) and the mixing fin (44b) create vortices that promote mixing of a contents of the fluid reservoir (28b), when the fluid collection cartridge (20b) is rotated about a longitudinal axis thereof.

8. The fluid collection system of any of claims 1-7, further comprising a fluid treatment material (70b) applied so as to be within the internal reservoir (28b), the fluid treatment material (70b) comprising an anticoagulant, clotting agent, or stabilization additive.

9. The fluid collection system of any of claims 1-8, further comprising a luer adapter (80b) insertable through the pierceable closure (40b).

10. The fluid collection system of claim 1, wherein the fluid collection cartridge (20b) is insertable into a proximal end of the holder (13b), with the fluid collection cartridge (20b) advanceable distally through the holder (13b) until the pointed proximal end (51b) of the needle cannula (50b) penetrates the pierceable closure (40b) and the lumen (53b) is in fluid communication with the internal reservoir (28b) and the fluid treatment material (70b) therein.

11. The fluid collection system of claim 10, wherein the needle assembly (11b) includes a first needle shield (60b) and a second needle shield (61b), with the first needle shield (60b) covering the distal end (52b) of the needle cannula (50b) and the second needle shield (61b) covering the proximal end (51b) of the needle cannula (50b).

12. The fluid collection system of claim 11, wherein the first needle shield (60b) includes an indicator tip (62b) that is activated by the presence of the fluid treatment material (70b).

13. The fluid collection system of any of claims 1-12, wherein the wherein the needle assembly (11b) further includes a multiple sample sleeve (57b) mounted over the proximal portion of the needle cannula (50b) and secured to a proximal end (55b) of the hub (54b), with the proximal portions of the needle cannula (50b) and the multiple sample sleeve (57b) projecting into the holder (13b) when the hub (54b) of the needle assembly (11b) is mounted to the holder (13b).

14. The fluid collection system of claim 1, wherein the outer shield (50c) further includes a pair of wings (68c) extending laterally from the outer shield (50c) at opposing sides thereof, to assist in positioning and placing the shieldable needle device (12c).

## Patentansprüche

1. Fluidentnahmesystem (10), das aufweist:
eine Fluidentnahmekartusche (20b), die aufweist:
ein rohrförmiges Element (21b) mit einem proximalen Ende (23b), einem offenen distalen Ende (22b) und einer Seitenwand (25b), die sich zwischen dem proximalen Ende und dem distalen Ende erstreckt und eine innere Kammer (26b) mit einem inneren Reservoir (28b) definiert;
einen durchstechbaren Verschluss (40b), der mit dem offenen distalen Ende des rohrförmigen Elements (21b) verbunden ist, wobei der Verschluss so ausgebildet ist, dass er mit der Seitenwand (25b) des rohrförmigen Elements (21b) zusammenwirkt, um das offene distale Ende (22b) abdichtend zu verschließen; und
eine Kolbenstangenbaugruppe (30b), die einen Stopfen (32b) und eine Kolbenstange (31b) aufweist, die durch eine Eingriffsanordnung (90b) lösbar miteinander verbunden sind, wobei die Eingriffsanordnung (90b) so ausgebildet ist, dass sie es der Kolbenstange (31b) ermöglicht, eine distal gerichtete Kraft auf den Stopfen (32b) auszuüben, und dass sie es ermöglicht, die Kolbenstange (31b) bei Ausübung einer proximal gerichteten Kraft von dem Stopfen (32b) und von dem rohrförmigen Element (21b) zu lösen;
eine Nadelbaugruppe (11b), die mit der Fluidentnahmekartusche (20b) verbunden ist, wobei die Nadelbaugruppe (11b) aufweist:
eine Nadelkanüle (50b) mit einem spitz zulaufenden proximalen Ende (51b), einem spitz zulaufenden distalen Ende (52b) und einem Lumen (53b), das sich zwischen dem proximalen Ende (51b) und dem distalen Ende (52b) erstreckt;
einen Ansatz (54b), in der die Nadelkanüle (50b) so montiert ist, dass das spitz zulaufende proximale Ende (51b) der Nadelkanüle (50b) proximal über den Ansatz (54b) hinausragt und das spitz zulaufende distale Ende (52b) der Nadelkanüle (50b) distal über den Ansatz (54b) hinausragt; und
einen Halter (13b), an dem der Ansatz (54b) befestigt ist, wobei der Halter (13b) so ausgebildet ist, dass er die Fluidentnahmekartusche (20b) verschiebbar aufnimmt;
**dadurch gekennzeichnet, dass** das Fluidentnahmesystem ferner eine abschirmbare Nadelvorrichtung (12c) aufweist, die mit der Nadelbaugruppe (11b) verbindbar ist und aufweist:
einen Ansatz (30c);
eine Nadelkanüle (20c), die in einem inneren Durchgang des Ansatzes (30c) positioniert ist und von diesem gehalten wird, wobei sich die Nadelkanüle (20c) distal von dem Ansatz (30c) befindet;
ein flexibles Rohr (14c), das mit dem Ansatz (30c) verbindbar ist, um sich proximal von diesem zu erstrecken;
eine Halterung (16c), die an dem flexiblen Rohr (14c) auf einer dem Ansatz (30c) entgegengesetzten Seite montiert ist; und
eine äußere Abschirmung (50c), die zwischen einer eingefahrenen Position, in der eine Punktionsspitze (28c) der Nadelkanüle (20c) von einem distalen Ende der äußeren Abschirmung (50c) aus freiliegt, und einer ausgefahrenen Position, in der die Punktionsspitze (28c) von der äußeren Abschirmung (50c) bedeckt ist, bewegbar ist;
wobei
der Ansatz (30c) ferner eine erste Lasche (40c) aufweist, die sich von einer Außenfläche davon an einer Stelle angrenzend an ein proximales Ende des Ansatzes (30c) nach außen erstreckt, und wobei die äußere Abschirmung (50c) eine zweite Lasche (62c) aufweist, die sich davon nach außen erstreckt, wobei die erste Lasche (40c) und die zweite Lasche (62c) so ausgebildet sind, dass entgegengesetzte Kräfte, die auf die erste Lasche (40c) und die zweite Lasche (62c) ausgeübt werden, bewirken, dass sich die äußere Abschirmung (50c) in Richtung eines distalen Endes der Nadelkanüle (20c) aus der eingefahrenen Position in die ausgefahrene Position bewegt.

2. Fluidentnahmesystem nach Anspruch 1, wobei der Stopfen (32b) in verschiebbarem Kontakt mit einer Innenfläche (27b) der Seitenwand (25b) des rohrförmigen Elements (21b) steht und eine fluiddichte Abdichtung dazwischen bildet, so dass eine Probe innerhalb des inneren Reservoirs (28b) gehalten werden kann, wobei das innere Reservoir (28b) innerhalb der Kammer (26b) zwischen dem distalen Ende (22b) des rohrförmigen Elements (21b) und einer distalen Fläche (34b) des Stopfens (32b) ausgebildet ist.

3. Fluidentnahmesystem nach Anspruch 1 oder Anspruch 2, wobei der Stopfen (32b) ein Stopfen mit geringem Widerstand ist, so dass das Vorhandensein von Fluiddruck innerhalb des inneren Reservoirs (28b) den Stopfen (32b) dazu veranlasst, in einer proximalen Richtung zum proximalen Ende (23b) des rohrförmigen Elements (21b) zu gleiten, bis eine proximale Fläche (35b) des Stopfens (32b) einen ringförmigen Flansch (24b) am proximalen Ende des rohrförmigen Elements (21b) kontaktiert.

4. Fluidentnahmesystem nach Anspruch 3, wobei der Stopfen (32b) ein selbstschmierender Stopfen ist.

5. Fluidentnahmesystem nach einem der Ansprüche 1-4, wobei der durchstechbare Verschluss (40b) so ausgebildet ist, dass er von einer Nadel (50b) durchstochen werden kann, um eine biologische Probe in das rohrförmige Element (21b) einzuführen, wobei der durchstechbare Verschluss (40b) so ausgebildet ist, dass er sich nach dem Entfernen der Nadel (50b) wieder verschließt.

6. Fluidentnahmesystem nach einem der Ansprüche 1-5, wobei der durchstechbare Verschluss (40b) einen Hohlraum (43b) definiert, der sich in ein inneres Ende (42b) des durchstechbaren Verschlusses (40b) erstreckt, und wobei eine distale Fläche (34b) des Stopfens (32b) eine Mischrippe (44b) aufweist, die sich distal davon erstreckt und so bemessen ist, dass sie in dem Hohlraum (43b) aufgenommen werden kann.

7. Fluidentnahmesystem nach Anspruch 6, wobei der Hohlraum (43b) und die Mischrippe (44b) Wirbel erzeugen, die das Mischen eines Inhalts des Fluidreservoirs (28b) fördern, wenn die Fluidentnahmekartusche (20b) um ihre Längsachse gedreht wird.

8. Fluidentnahmesystem nach einem der Ansprüche 1-7, das ferner ein Fluidbehandlungsmaterial (70b) aufweist, das so aufgebracht wird, dass es sich innerhalb des inneren Reservoirs (28b) befindet, wobei das Fluidbehandlungsmaterial (70b) ein Antikoagulans, ein Gerinnungsmittel oder ein Stabilisierungsadditiv umfasst.

9. Fluidentnahmesystem nach einem der Ansprüche 1-8, das außerdem einen Luer-Adapter (80b) aufweist, der durch den durchstechbaren Verschluss (40b) einführbar ist.

10. Fluidentnahmesystem nach Anspruch 1, wobei die Fluidentnahmekartusche (20b) in ein proximales Ende des Halters (13b) einführbar ist, wobei die Fluidentnahmekartusche (20b) distal durch den Halter (13b) vorschiebbar ist, bis das spitz zulaufende proximale Ende (51b) der Nadelkanüle (50b) den durchstechbaren Verschluss (40b) durchdringt und das Lumen (53b) in Fluidverbindung mit dem inneren Reservoir (28b) und dem darin befindlichen Fluidbehandlungsmaterial (70b) steht.

11. Fluidentnahmesystem nach Anspruch 10, wobei die Nadelbaugruppe (11b) eine erste Nadelabschirmung (60b) und eine zweiten Nadelabschirmung (61b) aufweist, wobei die erste Nadelabschirmung (60b) das distale Ende (52b) der Nadelkanüle (50b) bedeckt und die zweite Nadelabschirmung (61b) das proximale Ende (51b) der Nadelkanüle (50b) bedeckt.

12. Fluidentnahmesystem nach Anspruch 11, wobei die erste Nadelabschirmung (60b) eine Indikatorspitze (62b) aufweist, die durch das Vorhandensein des Fluidbehandlungsmaterials (70b) aktiviert wird.

13. Fluidentnahmesystem nach einem der Ansprüche 1-12, wobei die Nadelbaugruppe (11b) ferner eine Mehrfachprobenhülse (57b) aufweist, die über dem proximalen Abschnitt der Nadelkanüle (50b) montiert und an einem proximalen Ende (55b) des Ansatzes (54b) befestigt ist, wobei die proximalen Abschnitte der Nadelkanüle (50b) und die Mehrfachprobenhülse (57b) in den Halter (13b) hineinragen, wenn der Ansatz (54b) der Nadelbaugruppe (11b) an dem Halter (13b) montiert ist.

14. Fluidentnahmesystem nach Anspruch 1, wobei die äußere Abschirmung (50c) ferner ein Paar von Flügeln (68c) aufweist, die sich seitlich von der äußeren Abschirmung (50c) an deren entgegengesetzten Seiten erstrecken, um die Positionierung und Platzierung der abschirmbaren Nadelvorrichtung (12c) zu unterstützen.

## Revendications

1. Système de prélèvement de fluide (10) comprenant :
une cartouche de prélèvement de fluide (20b) comprenant :
un élément tubulaire (21b) ayant une extrémité proximale (23b), une extrémité distale ouverte (22b), et une paroi latérale (25b) s'étendant entre l'extrémité proximale et l'extrémité distale et définissant une chambre interne (26b) ayant un réservoir interne (28b) ;
une fermeture pouvant être percée (40b) associée à l'extrémité distale ouverte de l'élément tubulaire (21b), la fermeture étant configurée pour coopérer avec la paroi latérale (25b) de l'élément tubulaire (21b) afin de fermer de manière étanche ladite extrémité distale ouverte (22b) ; et
un ensemble tige de piston (30b) comprenant un bouchon (32b) et une tige de piston (31b) associés de manière amovible entre eux par un agencement d'engagement mutuel (90b), où ledit agencement d'engagement mutuel (90b) est configuré pour permettre à la tige de piston (31b) d'appliquer une force dirigée de manière distale au bouchon (32b) et pour permettre le retrait de la tige de piston (31b) du bouchon (32b) et de l'élément tubulaire (21b) lors de l'application d'une force dirigée de manière proximale ;
un ensemble aiguille (11b) relié à la cartouche de prélèvement de fluide (20b), l'ensemble aiguille (11b) comprenant :
une canule d'aiguille (50b) ayant une extrémité proximale pointue (51b), une extrémité distale pointue (52b) et une lumière (53b) s'étendant entre l'extrémité proximale (51b) et l'extrémité distale (52b) ;
un raccord (54b) dans lequel la canule d'aiguille (50b) est montée de sorte que l'extrémité proximale pointue (51b) de la canule d'aiguille (50b) fasse saillie de manière proximale au-delà du raccord (54b) et que l'extrémité distale pointue (52b) de la canule d'aiguille (50b) fasse saillie de manière distale au-delà du raccord (54b) ; et
un support (13b) auquel le raccord (54b) est fixé, le support (13b) étant configuré pour recevoir en coulissement la cartouche de prélèvement de fluide (20b) ; **caractérisé en ce que** le système de prélèvement de fluide comprend en outre
un dispositif à aiguille pouvant être protégé (12c) pouvant être relié à l'ensemble aiguille (11b), comprenant :
un raccord (30c) ;
une canule d'aiguille (20c) positionnée dans et supportée par un passage interne du raccord (30c), la canule d'aiguille (20c) étant distale par rapport au raccord (30c) ;
un tube flexible (14c) pouvant être relié au raccord (30c) pour s'étendre de manière proximale depuis celui-ci ;
un dispositif de fixation (16c) monté sur le tube flexible (14c), sur un côté de celui-ci opposé au raccord (30c) ; et
une protection externe (50c) mobile entre une position rétractée dans laquelle une pointe de ponction (28c) de la canule d'aiguille (20c) est exposée depuis une extrémité distale de la protection externe (50c), et une position étendue dans laquelle la pointe de ponction (28c) est recouverte par la protection externe (50c) ;
dans lequel
le raccord (30c) comprend en outre une première languette (40c) s'étendant vers l'extérieur depuis une surface externe de celui-ci à un emplacement adjacent à une extrémité proximale du raccord (30c), et où la protection externe (50c) comprend une seconde languette (62c) s'étendant vers l'extérieur depuis celui-ci, la première languette (40c) et la seconde languette (62c) étant configurées de sorte que des forces opposées appliquées contre la première languette (40c) et la seconde languette (62c) amènent la protection externe (50c) à se déplacer vers une extrémité distale de la canule d'aiguille (20c), de la position rétractée à la position étendue.

2. Système de prélèvement de fluide selon la revendication 1, dans lequel le bouchon (32b) est en contact coulissant avec une surface interne (27b) de la paroi latérale (25b) de l'élément tubulaire (21b) et fournit une étanchéité aux fluides entre eux, de sorte qu'un échantillon puisse être contenu dans le réservoir interne (28b), le réservoir interne (28b) étant formé dans la chambre (26b) entre l'extrémité distale (22b) de l'élément tubulaire (21b) et une face distale (34b) du bouchon (32b).

3. Système de prélèvement de fluide selon la revendication 1 ou 2, dans lequel le bouchon (32b) est un bouchon à faible résistance, de sorte que la présence de pression de fluide dans le réservoir interne (28b) amène le bouchon (32b) à coulisser dans une direction proximale vers l'extrémité proximale (23b) de l'élément tubulaire (21b) jusqu'à ce qu'une face proximale (35b) du bouchon (32b) entre en contact avec une bride annulaire (24b) au niveau de l'extrémité proximale de l'élément tubulaire (21b).

4. Système de prélèvement de fluide selon la revendication 3, dans lequel le bouchon (32b) est un bouchon autolubrifiant.

5. Système de prélèvement de fluide selon l'une des revendications 1 à 4, dans lequel la fermeture pouvant être percée (40b) est configurée pour être percée par une aiguille (50b) afin d'introduire un échantillon biologique dans l'élément tubulaire (21b), la fermeture pouvant être percée (40b) étant configurée pour se resceller après le retrait de l'aiguille (50b).

6. Système de prélèvement de fluide selon l'une des revendications 1 à 5, dans lequel la fermeture pouvant être percée (40b) définit une cavité (43b) s'étendant dans une extrémité interne (42b) de la fermeture pouvant être percée (40b), et dans lequel une face distale (34b) du bouchon (32b) comprend une ailette de mélange (44b) s'étendant de manière distale depuis celle-ci qui est dimensionnée pour être reçue dans la cavité (43b).

7. Système de prélèvement de fluide selon la revendication 6, dans lequel la cavité (43b) et l'ailette de mélange (44b) créent des tourbillons qui favorisent le mélange du contenu du réservoir de fluide (28b), lorsque la cartouche de prélèvement de fluide (20b) tourne autour d'un axe longitudinal de celle-ci.

8. Système de prélèvement de fluide selon l'une des revendications 1 à 7, comprenant en outre un matériau de traitement de fluide (70b) appliqué de manière à se trouver dans le réservoir interne (28b), le matériau de traitement de fluide (70b) comprenant un anticoagulant, un agent de coagulation ou un additif de stabilisation.

9. Système de prélèvement de fluide selon l'une des revendications 1 à 8, comprenant en outre un adaptateur Luer (80b) pouvant être inséré à travers la fermeture pouvant être percée (40b).

10. Système de prélèvement de fluide selon la revendication 1, dans lequel la cartouche de prélèvement de fluide (20b) peut être insérée dans une extrémité proximale du support (13b), la cartouche de prélèvement de fluide (20b) pouvant avancer de manière distale à travers le support (13b) jusqu'à ce que l'extrémité proximale pointue (51b) de la canule d'aiguille (50b) pénètre dans la fermeture pouvant être percée (40b) et que la lumière (53b) soit en communication fluidique avec le réservoir interne (28b) et le matériau de traitement de fluide (70b) dedans.

11. Système de prélèvement de fluide selon la revendication 10, dans lequel l'ensemble aiguille (11b) comprend une première protection d'aiguille (60b) et une seconde protection d'aiguille (61b), la première protection d'aiguille (60b) recouvrant l'extrémité distale (52b) de la canule d'aiguille (50b) et la seconde protection d'aiguille (61b) recouvrant l'extrémité proximale (51b) de la canule d'aiguille (50b).

12. Système de prélèvement de fluide selon la revendication 11, dans lequel la première protection d'aiguille (60b) comprend une pointe d'indication (62b) qui est activée par la présence du matériau de traitement de fluide (70b).

13. Système de prélèvement de fluide selon l'une des revendications 1 à 12, dans lequel l'ensemble aiguille (11b) comprend en outre un manchon à échantillons multiples (57b) monté sur la partie proximale de la canule d'aiguille (50b) et fixé à une extrémité proximale (55b) du raccord (54b), les parties proximales de la canule d'aiguille (50b) et du manchon à échantillons multiples (57b) faisant saillie dans le support (13b) lorsque le raccord (54b) de l'ensemble aiguille (11b) est monté sur le support (13b).

14. Système de prélèvement de fluide selon la revendication 1, dans lequel la protection externe (50c) comprend en outre une paire d'ailes (68c) s'étendant latéralement depuis la protection externe (50c) à des côtés opposés de celui-ci, pour aider à positionner et à placer le dispositif à aiguille pouvant être protégé (12c).
